# EUROPEAN PATENT SPECIFICATION

(11) **EP 3 217 998 B1**
(45) Date of publication and mention of the grant of the patent: **23.03.2022**
(21) Application number: 15858766.7
(22) Date of filing: 11.11.2015
(51) Int. Cl.: A61K 9/00, A61K 38/17, A61P 25/00

(54) **APOAEQUORIN-CONTAINING COMPOSITIONS FOR USE IN A METHOD FOR THE TREATMENT OF NEURONAL CELL DEATH FROM ISCHEMIA BY ORAL ADMINISTRATION**
APOAEQUORINHALTIGE ZUSAMMENSETZUNGEN ZUR VERWENDUNG IN EINEM VERFAHREN ZUR BEHANDLUNG DES NEURONALEN ZELLTODES DURCH ISCHÄMIE DURCH ORALE VERABREICHUING
COMPOSITIONS CONTENANT DE L'APOAÉQUORINE POUR UTILISATION DANS UNE MÉTHODE DE TRAITEMENT DE LA MORT DES CELLULES NEURONALES PAR ISCHÉMIE PAR ADMINISTRATION ORALE

(30) Priority: 11.11.2014 US 201462078099 P
(43) Date of publication of application: 20.09.2017
(73) Proprietor: Quincy Bioscience LLC, Madison, WI 53717 (US)
(72) Inventor: UNDERWOOD, Mark Y., Verona, WI 53593 (US); MOYER, James R. Jr., Mequon, WI 53097 (US)
(74) Representative: J A Kemp LLP
(86) International application number: PCT/US2015/060116
(87) International publication number: WO 2016/077437

(56) References cited:
- WO-A1-2013/074798
- US-A1- 2011 124 562
- US-A1- 2011 130 336
- DATABASE BIOSIS BIOSCIENCES INFORMATION SERVICE, PHILADELPHIA, PA, US; 17 October 2012 (2012-10-17), Hochstetter E. L.: "Apoaequorin", XP009166932,
- DETERT ET AL.: 'Pretreatment with Apoaequorin Protects Hippocampal CA 1 Neurons from Oxygen-Glucose Deprivation' PLOS ONE vol. 8, no. 11, 01 November 2013, pages 1 - 10, XP055425497 DOI: 10.1371/JOURNAL.PONE.0079002
- None

## Description

### FIELD OF THE INVENTION

This invention relates generally to compositions useful for the treatment of neuronal inflammation. More specifically, the present invention is directed to apoaequorin for use in treating neuronal inflammation.

### BACKGROUND OF THE INVENTION

In 2009, stroke accounted for about one of every 19 deaths in the United States, making it the third leading cause of death behind only heart disease and cancer. As a result, finding ways to ameliorate injury following stroke is imperative. Much attention has been placed on the role of calcium in ischemia and possible neuroprotection by blocking its toxic effects *post*-ischemia.

Calcium (Ca²⁺) plays a pivotal role in various neuronal processes, including neurotransmitter release and synaptic plasticity. Neurons are continuously subjected to fluctuations in intracellular Ca²⁺ as a result of ongoing activity, however excess or sustained increases in intracellular Ca²⁺ can be toxic to neurons. Thus, neuronal intracellular Ca²⁺ is very tightly regulated, and several mechanisms exist which enable neurons to limit or control cytosolic Ca²⁺ levels. In particular, calcium binding proteins (CaBPs; such as calbindin, parvalbumin, and calretinin) are important for binding and buffering cytosolic Ca²⁺.

Studies in the hippocampus have shown that the presence of CaBPs confers some protection against excitotoxic insults that normally result in cell death. Interestingly, decreased levels of CaBPs are observed with advancing age, and in neurodegenerative disorders, including Alzheimer's disease, and Parkinson's disease. Treatments aimed at minimizing Ca²⁺ toxicity during ischemia by administering CaBPs before an ischemic insult have also had positive results. For example, Yenari et al. treated animals with calbindin prior to inducing ischemia and found that over-expression of calbindin was neuroprotective. In addition, Fan et al. treated rats with calbindin prior to ischemia and demonstrated a smaller infarct volume, better behavioral recovery, and decreased apoptosis in the calbindin-treated animals. Indeed, much research has focused on understanding the deleterious effects of stroke. Interestingly, a major risk factor for stroke is aging, and one prominent hypothesis of brain aging is the Ca²⁺ hypothesis of aging. This hypothesis argues that an aging-related change in the ability to regulate calcium and calcium-dependent processes is a critical contributor to an increase in susceptibility to cognitive decline and neurodegenerative disorders. Given these aging-related changes in Ca²⁺, and the critical role of Ca²⁺ in ischemic cell death, much research has focused on Ca²⁺ dysregulation in both neurons and glia.

Excessive intracellular Ca²⁺ accumulation following ischemia is known to potentiate cell death through excitotoxicity. Following an ischemic insult, Ca²⁺ accumulates within the cell through voltage-gated Ca²⁺ channels (VGCCs), through NMDA receptors, and through release from intracellular organelles. Numerous studies have shown that blocking Ca²⁺ entry through NMDA receptors, VGCCs, or both in combination can be neuroprotective against ischemia. Interestingly, when NMDA receptor blockers were brought to clinical trials, they failed to provide neuroprotection and they produced undesirable side effects, such as hallucinations and coma. While it is uncertain why NMDA receptor blockers failed in clinical trials, it is clear that there is a need for continued research focused on ameliorating the devastating effects of ischemic stroke.

Despite advances, there is still a need for new and alternative therapeutics which treat neuronal inflammation. In particular, pharmaceutical or nutraceutical compositions which have reduced side effects as compared to prior agents are desired and, if discovered, would meet a long felt need in the medical and nutritional health communities.

Hochstetter et al., (Soc of Neuroscience Meeting, October 2012, presentation abstract 860.03/J1) discloses that apoaeqorin protects neurons from ischemia and alters cytokine mRNA levels in rat hippocampus.

US 2011/0130336 discloses compositions containing apoaeqorin and methods for their use in treating ischemic injury, particularly reduction in neuronal cell death following ischemic insult.

WO2013/074798 discloses methods for preconditioning neurons in a subject to reduce neuronal cell death due to brain ischemia using compositions containing apoaeqorin.

US2011/0124562 discloses methods for treating symptoms & disorders related to calcium imbalances, such as sleep quality, mood quality, memory quality and pain using compositions containing apoaeqorin.

Detert et al., (PLOS One, 2013, vol 8, e79002) discloses pretreatment with apoaeqorin protects hippocampal neurons from oxygen-glucose deprivation.

### SUMMARY OF THE INVENTION

The present invention is based in part on the inventors' recent research on apoaequorin, a calcium binding protein, and the unexpected finding that apoaequorin possesses novel neuroprotective abilities. In particular, apoaequorin has been found to be useful in preconditioning neurons in a subject to reduce neuronal cell death following ischemia.

In a first aspect, the present invention is directed to apoaequorin for use in a method of therapy comprising preconditioning neurons to reduce neuronal cell death following ischemia in a subject susceptible to ischemic cell death, said apoaequorin provided in a therapeutically effective dosage to precondition neurons in the subject to reduce neuronal cell death and wherein said apoaequorin is administered orally in the form of a nutraceutical composition.

In one embodiment, the apoaequorin is in a unit dosage form selected from a tablet or capsule.

The present invention provides various advantages over prior compositions and methods in that it provides for the general improvement of a subject's mental and physical health through its neuroprotective functions.

Other objects, features and advantages of the present invention will become apparent after review of the specification and claims.

### BRIEF DESCRIPTION OF THE DRAWINGS

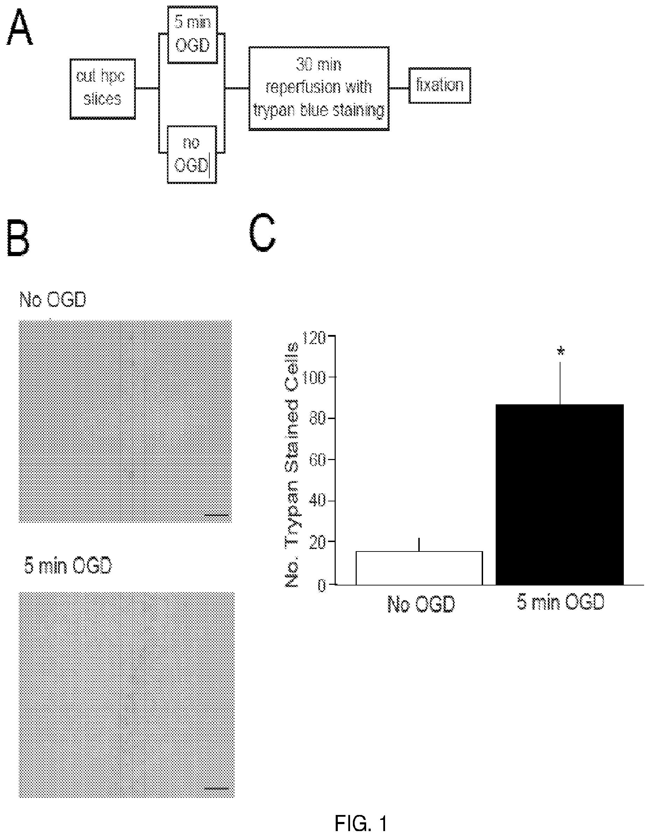

Fig. 1A-C depicts effects of oxygen-glucose deprivation on cell death in acute hippocampal brain slices. A) Diagram of experimental design. Coronal hippocampal slices were incubated for 1 hr in artificial cerebral spinal fluid (aCSF). Half of the slices were transferred to the ischemic condition for 5 min of oxygen-glucose deprivation (OGD) while the other half remained normoxic (no OGD). All of the slices were then transferred to aCSF for a 30 min reperfusion and trypan blue staining. The slices were then fixed in 10% neutral-buffered formalin. *B*) Representative images of trypan blue staining in area CA1 of the hippocampus in a slice that remained normoxic (no OGD) and in a slice subjected to 5 min OGD. Notice that there is less staining in the normoxic slice compared to the OGD slice. *C*) There was a significant increase in the number of trypan blue-stained neurons in area CA1 of the hippocampus from slices that underwent 5 min OGD compared to slices that remained normoxic (^{∗}*, p* < .01).

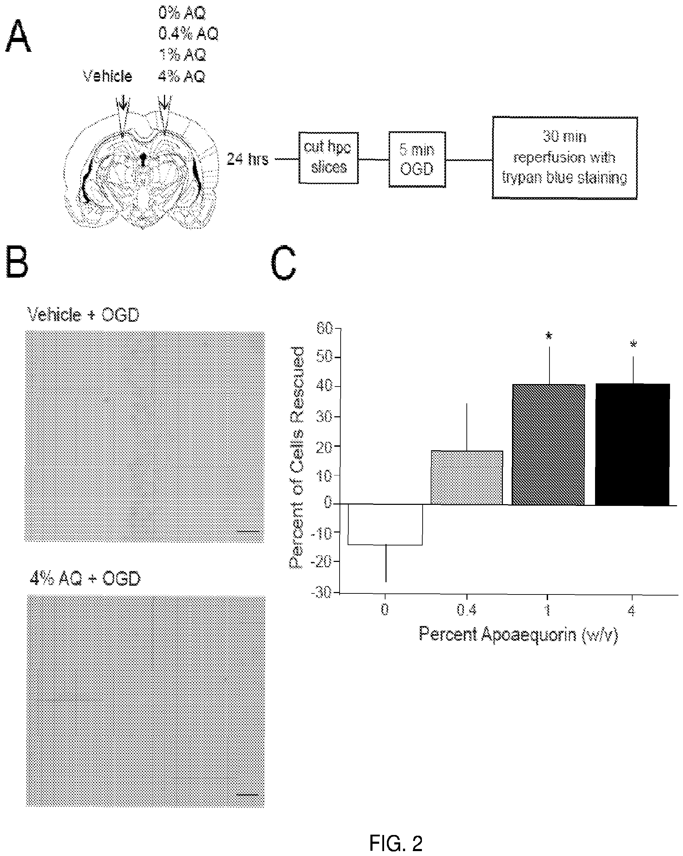

Fig. 2A-C depicts dose-dependent effects of apoaequorin on ischemic cell death. *A*) Diagram of experimental design. Rats that were cannulated bilaterally in the dorsal hippocampus were given an infusion of 0, 0.4, 1, or 4% apoaequorin (AQ) in one hemisphere and vehicle (0% AQ) in the other hemisphere. One day following the infusion, coronal hippocampal slices were cut and incubated in artificial cerebral spinal fluid (aCSF) for 1 hr. All slices were transferred to the ischemic condition for 5 min of oxygen-glucose deprivation (OGD). Slices were then transferred to aCSF for a 30 min reperfusion and trypan blue staining. The slices were then fixed in 10% neutral-buffered formalin. *B*) Representative images of trypan blue staining in area CA1 of the hippocampus following ischemia in a vehicle-treated slice or a 4% AQ-treated slice. Notice that there is less staining in the AQ-treated slice compared to the vehicle-treated slice. C) Graph shows neuroprotection (percent of cells rescued) as a function of the dose of apoaequorin. There was significant neuroprotection in the rats treated with 1 or 4% AQ (but not 0.4% AQ) compared to the 0% AQ (vehicle; ^{∗}*, p* < .01).

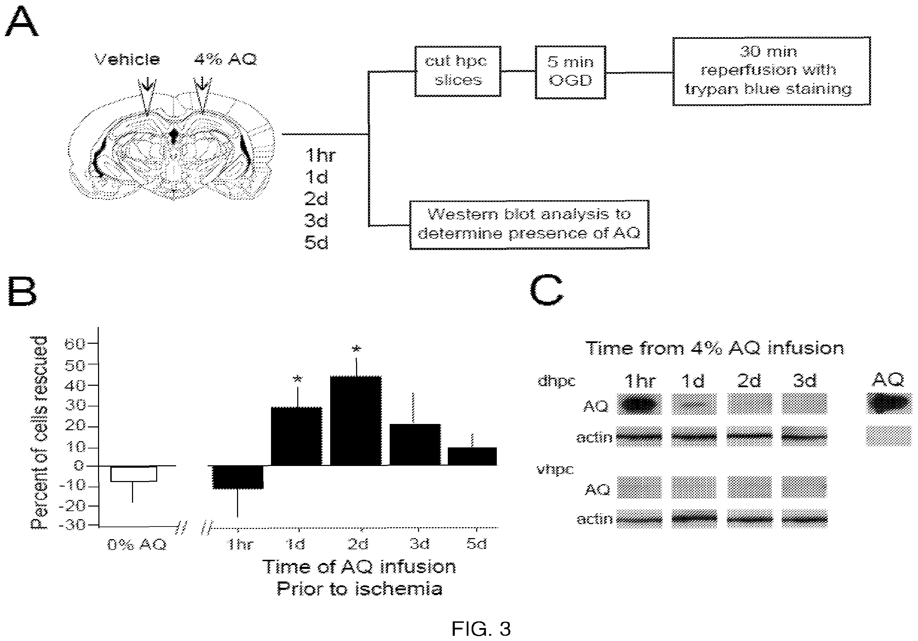

Fig. 3A-C depicts time-dependent effects of apoaequorin on ischemic cell death. *A*) Diagram of experimental design. Rats that were cannulated bilaterally in the dorsal hippocampus were given an infusion 4% apoaequorin (AQ) in one hemisphere and vehicle (0% AQ) in the other hemisphere. Coronal hippocampal slices were cut 1 hr, 1 day, 2 days, 3 days, or 5 days post-infusion, and slices were incubated for 1 hr in artificial cerebral spinal fluid (aCSF). All slices were transferred to the ischemic condition for a 5 min oxygen-glucose deprivation (OGD). Slices were then transferred to aCSF for a 30 min reperfusion and trypan blue staining. The slices were then fixed in 10% neutral-buffered formalin. A second set of rats was given bilateral infusion of 4% AQ and the brains were removed at 1 hr, 1 day, 2 days, or 3 days post-infusion to be used for Western blotting. *B*) An infusion of 4% AQ 1 or 2 days prior to ischemia resulted in significant neuroprotection, but the neuroprotective effect was no longer evident at 3 or 5 days post-infusion. Notice that AQ is also not neuroprotective when infused just 1 hr prior to ischemia (*p* = .78). *C*). Western blot analysis of the AQ protein at 22 kD. AQ is present in the dorsal hippocampus (AQ-dhpc) at 1 hr and 1 day, but is no longer present at 3 days post-infusion. At 2 days post-infusion, a band is present in only 29% of the rats. Notice that there is never a band in the ventral hippocampus (AQ-vhpc), regardless of the infusion time. Analysis of β-actin (45 kD) revealed no effect of protein loading at any time point in either dorsal (actin-dhpc) or ventral (actin-vhpc) hippocampus. ^{∗}*, p* < .01

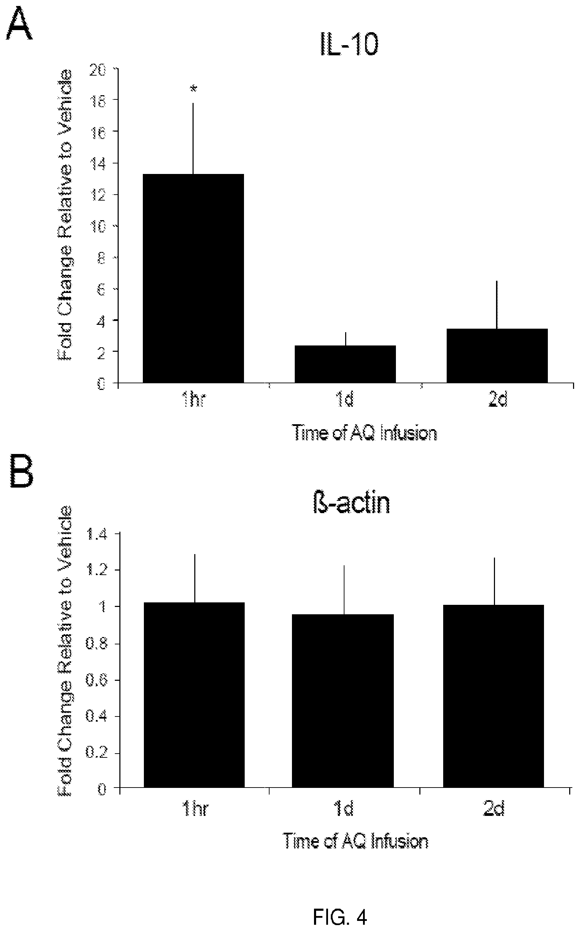

Fig. 4A-B depicts effects of apoaequorin on interleukin-10 mRNA expression. A) Interleukin-10 (IL-10) mRNA expression is significantly increased 1 hour after 4% AQ was infused into the dorsal hippocampus. This statistically significant increase was transient as IL-10 mRNA expression returned to near baseline levels within 1 to 2 days, although a biologically relevant 2- to 3-fold increase was still observed. *B*) β-actin mRNA expression did not significantly differ between 4% AQ and the vehicle-treated hemisphere (*p* = .52). For both graphs, data are expressed as fold-change from the vehicle-treated control hemisphere.
Fig. 5 illustrates the experimental methodology utilized in Example 2.
Fig. 6A-B depicts data showing intrahippocampal infusion of AQ is neuroprotective.
Fig. 7A-D depicts cytokine expression after AQ infusion.
Fig. 8A-C illustrates data demonstrating oral administration of AQ is neuroprotective.
Fig. 9A-C depicts data showing AQ infusion alters IL-10 and TNF-α protein expression.
Fig. 10A-C illustrates AQ infusion and trace fear conditioning in aging rats.
Fig. 11A-C depicts oral administration of AQ is time- & dose-dependent.
Fig. 12 depicts the experimental methodology utilized in Example 4.
Fig. 13A-C depicts oral administration of AQ is neuroprotective.
Fig. 14A-D shows data demonstrating oral administration of AQ alters cytokine protein expression.
Fig. 15A-B depicts shows data demonstrating intrahippocampal infusion of AQ alters cytokine protein expression.
Fig. 16 illustrates data showing IL-10 nAb reverses AQ's neuroprotective effect.

### DETAILED DESCRIPTION OF THE INVENTION

### I. IN GENERAL

Before the present materials and methods are described, it is understood that this invention is not limited to the particular methodology, and materials described, as these may vary. It is also to be understood that the terminology used herein is for the purpose of describing particular embodiments only.

It must be noted that as used herein and in the appended claims, the singular forms "a", "an", and "the" include plural reference unless the context clearly dictates otherwise. As well, the terms "a" (or "an"), "one or more" and "at least one" can be used interchangeably herein. It is also to be noted that the terms "comprising", "including", and "having" can be used interchangeably.

Unless defined otherwise, all technical and scientific terms used herein have the same meanings as commonly understood by one of ordinary skill in the art to which this invention belongs.

***Animals.*** 92 male F344 adult rats were used. Rats were kept on a 14/10-hr day/night cycle with access to food and water *ad libitum.* Weight for each animal was recorded two times per week, as to account for significant weight increases and/or decreases.

***Drugs.*** Apoaequorin (AQ; Quincy Bioscience) was prepared in double deionized water at a concentration of 7.4%. Experimental groups in the dose dependent experiments (n = 18) received 0 (n = 4), 3.6 (n = 5), 48 (n = 4), 240 (n = 3), or 480 mg/kg of AQ mixed into their daily PB. For the remainder of the studies, rats (n = 73) received 48 mg/kg of AQ mixed into their daily PB. Animals were assigned to one of five groups; No AQ (n = 12), 1 hour AQ (n = 17), 1 day AQ (n = 15), 2 days AQ (n = 15), and 7 days AQ (n = 14. Rats received ¼ teaspoon of PB placed in a petri dish in the cage every day at a designated time. Petri dishes were not removed until all PB was consumed. Animals were weighed twice per week, as to maintain proper AQ dosage.

AQ for infusion studies was prepared as previously described (Detert et al., 2013). IL-10 neutralizing antibody (nAb) and its IgG control were prepared in sterile PBS. 0.5 ug was infused at a rate of 1 ul/min through 1 ul Hamilton Syringes.

***Oxygen-Glucose Deprivation.*** On the last day of administration, rats were allowed 1 hour after PB consumption for digestion, deeply anesthetized with isoflurane, and coronal slices (400 µm) of dorsal hippocampus (dhpc; Bregma ⁻3.14 - ⁻4.16; Paxinos & Watson, 1998) were prepared using standard procedures (Moyer & Brown, 2007). Following 1 hr slice recovery in aCSF, one hemisphere of each brain (counterbalanced) was subjected to *in vitro* ischemia by transferring slices to an oxygen-glucose deprivation chamber (glucose replaced with fructose and bubbled with 95% N₂ - 5% CO₂ instead of a 95% O₂ - 5% CO₂) for 5 min, while the other hemisphere remained in recovery. All slices were then placed into oxygenated aCSF containing 0.2% trypan blue for a 30 min reperfusion period. Trypan blue stains dead cells while leaving living cells unstained (DeRenzis & Schechtman, 1973). The slices were rinsed twice in oxygenated, room temperature aCSF then fixed in 10% neutral buffered formalin overnight in the refrigerator. Slices were then cryoprotected in 30% sucrose, sectioned on a cryostat (40 µm), and mounted onto subbed slides for cell counts.

***Cell Counts.*** Slices were examined under an Olympus microscope (equipped with a digital camera) at 10X, and pictures were taken (CellSens). Trypan blue stained neurons within CA1 (about an 800 µm section) were counted by an experimenter blind to experimental conditions. Statistical analyses were performed using SPSS (v 21.0.0; IBM Corporation; Armonk, NY). An ANOVA was used to evaluate a drug effect, and Fisher's LSD post-hoc evaluations were used to evaluate group interactions. Asterisk (^{∗}) indicates p < .05.

***Western Blots.*** Animals were deeply anesthetized with isoflurane, brains rapidly removed, frozen, and stored at -80°C. Upon time of dissection, samples were dissected from dhpc (Bregma ⁻3.14 - ⁻4.16mm). Samples were homogenized, centrifuged at 4000 RPM for 20 min, supernatant was removed, and protein was measured using a Bradford protein assay kit (Bio-Rad). Protein samples were normalized and loaded for SDS-PAGE (12%). Proteins (30µg) were transferred onto PVDF membranes using the Turbo Transfer System (Bio-Rad). Membranes were incubated in blocking buffer (2 hr), primary antibody (overnight at 4 °C; 1:1000 mouse anti-aequorin [Chemicon] or 1:1000 rabbit anti-β-actin [Cell Signaling Technology], and secondary antibody (90 min; 1:20,000 goat anti-mouse [Santa Cruz Biotechnology] or 1:40,000 goat anti-rabbit [Millipore]). Membranes were then washed, placed in a chemiluminescence solution (Thermo Scientific), and imaged with a Syngene GBox. Images were taken with GeneSys software (v 1.2.4.0; Synoptics camera 4.2MP), and fluorescence for each band was evaluated with GeneTools software (v 4.02; Cambridge, England). Values are expressed as a percentage of control animals. Statistics were performed with SPSS (v. 21).

Although any methods and materials similar or equivalent to those described herein can be used in the practice or testing of the present invention, the preferred methods and materials are now described. All references cited in this specification are to be taken as indicative of the level of skill in the art. Nothing herein is to be construed as an admission that the invention is not entitled to antedate such disclosure by virtue of prior invention.

### II. THE INVENTION

Ischemic stroke affects ~795,000 people each year in the U.S., which results in an estimated annual cost of $73.7 billion. Calcium is pivotal in a variety of neuronal signaling cascades, however, during ischemia, excess calcium influx can trigger excitotoxic cell death. Calcium binding proteins help neurons regulate/buffer intracellular calcium levels during ischemia. Aequorin is a calcium binding protein isolated from the jellyfish *Aequorea victoria,* and has been used for years as a calcium indicator, but little is known about its neuroprotective properties. The present study used an *in vitro* rat brain slice preparation to test the hypothesis that an intra-hippocampal infusion of apoaequorin (the calcium binding component of aequorin) protects neurons from ischemic cell death. Bilaterally cannulated rats received an apoaequorin infusion in one hemisphere and vehicle control in the other. Hippocampal slices were then prepared and subjected to 5 minutes of oxygen-glucose deprivation (OGD), and cell death was assayed by trypan blue exclusion. Apoaequorin dose-dependently protected neurons from OGD - doses of 1% and 4% (but not 0.4%) significantly decreased the number of trypan blue-labeled neurons. This effect was also time dependent, lasting up to 48 hours. This time dependent effect was paralleled by changes in cytokine and chemokine expression, indicating that apoaequorin may protect neurons via a neuroimmunomodulatory mechanism. These data support the hypothesis that pretreatment with apoaequorin protects neurons against ischemic cell death, and may be an effective neurotherapeutic.

Aequorin is a photo-protein originally isolated from luminescent jellyfish and other marine organisms. The aequorin complex comprises a 22,285-dalton apoaequorin protein, molecular oxygen and the luminophore coelenterazine. When three Ca²⁺ ions bind to this complex, coelenterazine is oxidized to coelentermide, with a concomitant release of carbon dioxide and blue light. Aequorin is not exported or secreted by cells, nor is it compartmentalized or sequestered within cells. Accordingly, aequorin measurements have been used to detect Ca²⁺ changes that occur over relatively long periods. In several experimental systems, aequorin's luminescence was detectable many hours to days after cell loading. It is further known that aequorin also does not disrupt cell functions or embryo development.

Because of its Ca²⁺-dependent luminescence, the aequorin complex has been extensively used as an intracellular Ca²⁺ indicator. *Aequorea victoria* aequorin has been specifically used to: (1) analyze the secretion response of single adrenal chromaffin cells to nicotinic cholinergic agonists; (2) clarify the role of Ca²⁺ release in heart muscle damage; (3) demonstrate the massive release of Ca²⁺ during fertilization; (4) study the regulation of the sarcoplasmic reticulum Ca²⁺ pump expression in developing chick myoblasts; and (5) calibrate micropipets with injection volumes of as little as three picoliters.

Apoaequorin has an approximate molecular weight of 22 kDa. Apoaequorin can be used to regenerate aequorin by reducing the disulfide bond in apoaequorin. The calcium-loaded apoaequorin retains the same compact scaffold and overall folding pattern as unreacted photoproteins containing a bound substrate.

Conventional purification of aequorin from the jellyfish *Aequorea victoria* requires laborious extraction procedures and sometimes yields preparations that are substantially heterogeneous or that are toxic to the organisms under study. Two tons of jellyfish typically yield approximately 125mg of the purified photoprotein. In contrast, recombinant aequorin is preferably produced by purifying apoaequorin from genetically engineered *Escherichia coli,* followed by reconstitution of the aequorin complex *in vitro* with pure coelenterazine. Apoaequorin useful in the present invention has been described and is commercially-obtainable through purification schemes and/or syntheses known to those of skill in the art. S. Inouye, S. Zenno, Y. Sakaki, and F. Tsuji. High level expression and purification of apoaequorin. (1991) Protein Expression and Purification 2, 122-126.

Aequorin is a CaBP isolated from the coelenterate *Aequorea victoria.* Aequorin belongs to the EF-hand family of CaBPs, with EF-hand loops that are closely related to CaBPs in mammals. In addition, aequorin has been used for years as an indicator of Ca²⁺ and has been shown to be safe and well tolerated by cells. However, to date, no studies have investigated its therapeutic potential. Aequorin is made up of two components - the calcium binding component apoaequorin (AQ) and the chemiluminescent molecule coelenterazine. Since the AQ portion of this protein contains the calcium binding domains, AQ was used in the present studies.

For the current experiments, we used an *in vitro* model of global ischemia in acute hippocampal brain slices. In acute hippocampal slices, OGD-induced damage is most evident in area CA1 of the hippocampus, similar to that seen *in vivo.* Acute hippocampal slices offer many advantages over use of cell cultures and *in vivo* models, including that the tissue morphology is relatively unchanged from the intact animal, changes in extracellular ion concentration and release of neurotransmitters are similar to that reported *in vivo,* and there is no vascular or other systemic responses that cannot be controlled *in vivo.* Neuronal damage following OGD in acute slices is seen within the first 30 minutes of reperfusion, however, due to the short life of slices, only early changes in ischemia are able to be analyzed. Because hippocampal neurons are vulnerable to cell death following ischemia, we tested the hypothesis that an infusion of AQ directly into the hippocampus will be neuroprotective when administered prior to an ischemic insult.

The administration of apoaequorin-containing compositions to a subject may be used to correct or maintain the calcium balance in that subject. The maintenance of ionic calcium concentrations in plasma and body fluids is understood to be critical to a wide variety of bodily functions, including, but not limited to neuronal excitability, muscle contraction, membrane permeability, cell division, hormone secretion, bone mineralization, or the prevention of cell death following ischemia. Disruption in calcium homeostasis, i.e., a calcium imbalance, is understood to cause and/or correlate with many diseases, syndromes and conditions. Exemplary diseases, syndromes and conditions include those associated with sleep quality, energy quality, mood quality, memory quality and pain perception. The study of CaBPs has led to their recognition as protective factors acting in the maintenance of proper ionic calcium levels.

In certain embodiments, apoaequorin may be administered as the sole active ingredient for providing neuroprotection, for delaying the progression of neuronal inflammation, for preventing the onset of neuronal inflammation, and for preventing and/or treating the recurrence of neuronal inflammation. In certain embodiments, apoaequorin may be administered in combination with one or more additional agents having known therapeutic or nutraceutical value.

As used herein, the term "treating" includes preventative as well as disorder remittent treatment. As used herein, the terms "reducing", "alleviating", "suppressing" and "inhibiting" have their commonly understood meaning of lessening or decreasing. As used herein, the term "progression" means increasing in scope or severity, advancing, growing or becoming worse. As used herein, the term "recurrence" means the return of a disease after a remission.

As used herein, the term "administering" refers to bringing a patient, tissue, organ or cell in contact with apoaequorin. As used herein, administration can be accomplished *in vitro,* i.e., in a test tube, or *in vivo,* i.e., in cells or tissues of living organisms, for example, humans. A "patient" or "subject", used equivalently herein, refers to a mammal, preferably a human, that either: (1) has neuronal inflammation remediable or treatable by administration of apoaequorin; or (2) is susceptible to a neuronal inflammation that is preventable by administering apoaequorin.

As used herein, the terms "effective amount" and "therapeutically effective amount" refer to the quantity of active agents sufficient to yield a desired therapeutic response without undue adverse side effects such as toxicity, irritation, or allergic response. The specific "effective amount" will, obviously, vary with such factors as the particular condition being treated, the physical condition of the patient, the type of animal being treated, the duration of the treatment, the nature of concurrent therapy (if any), and the specific formulations employed and the structure of the compounds or its derivatives. In this case, an amount would be deemed therapeutically effective if it resulted in one or more of the following: (1) the prevention of neuronal inflammation; and (2) the reversal or stabilization of a neuronal inflammation. The optimum effective amounts can be readily determined by one of ordinary skill in the art using routine experimentation.

In certain preferred compositions for oral administration to subjects, apoaequorin is formulated with at least one acceptable carrier at a dosage of approximately 10 mg/dose, a dose preferably in capsule form, with recommended dosage for a subject approximately 10 mg/day (i.e., one capsule per day).

Compositions according to the present invention include liquids or lyophilized or otherwise dried formulations and include diluents of various buffer content (e.g., Tris-HCl, acetate, phosphate), pH and ionic strength, additives such as albumin or gelatin to prevent absorption to surfaces, detergents (e.g., Tween 20, Tween 80, Pluronic F68, bile acid salts), solubilizing agents (e.g., glycerol, polyethylene glycerol), antioxidants (e.g., ascorbic acid, sodium metabisulfite), preservatives (e.g., Thimerosal, benzyl alcohol, parabens), bulking substances or tonicity modifiers (e.g., lactose, mannitol), covalent attachment of polymers such as polyethylene glycol to the protein, complexation with metal ions, or incorporation of the material into or onto particulate preparations of polymeric compounds such as polylactic acid, polyglycolic acid, or hydrogels, or onto liposomes, microemulsions, micelles, lamellar or multilamellar vesicles, erythrocyte ghosts or spheroplasts. Such compositions will influence the physical state, solubility, stability, rate of *in vivo* release, and rate of *in vivo* clearance. Controlled or sustained release compositions include formulation in lipophilic depots (e.g., fatty acids, waxes, oils).

Also disclosed herein are particulate compositions coated with polymers (e.g., poloxamers or poloxamines). Other embodiments of the compositions incorporate particulate forms protective coatings, protease inhibitors or permeation enhancers for oral administration.

Further, as used herein, "pharmaceutically acceptable carriers" are well known to those skilled in the art and include, but are not limited to, 0.01-0.1M and preferably 0.05M phosphate buffer or 0.9% saline. Additionally, such pharmaceutically acceptable carriers may be aqueous or non-aqueous solutions, suspensions and emulsions. Examples of non-aqueous solvents are propylene glycol, polyethylene glycol, vegetable oils such as olive oil and injectable organic esters such as ethyl oleate. Aqueous carriers include water, alcoholic/aqueous solutions, emulsions or suspensions, including saline and buffered media.

Other embodiments of the compositions administered according to the invention incorporate particulate forms, protective coatings, protease inhibitors or permeation enhancers for oral administration.

Chemical entities modified by the covalent attachment of water-soluble polymers such as polyethylene glycol, copolymers of polyethylene glycol and polypropylene glycol, carboxymethyl cellulose, dextran, polyvinyl alcohol, polyvinylpyrrolidone or polyproline are known to exhibit substantially longer half-lives in blood following intravenous injection than do the corresponding unmodified compounds. Such modifications may also increase the chemical entities solubility in aqueous solution, eliminate aggregation, enhance the physical and chemical stability of the compound, and greatly reduce the immunogenicity and reactivity of the compound. As a result, the desired *in vivo* biological activity may be achieved by the administration of such polymer-entity abducts less frequently or in lower doses than with the unmodified entity.

The composition can comprise apoaequorin alone, or can further include a pharmaceutically acceptable carrier, and can be in solid or liquid form such as tablets, powders, capsules, pellets, solutions, suspensions, elixirs, syrups, beverages, emulsions, and gels.. Pharmaceutically acceptable carriers also include gums, starches, sugars, cellulosic materials, and mixtures thereof.

The compositions administrable by the invention can be prepared by known dissolving, mixing, granulating, or tabletforming processes. For oral administration, apoaequorin or its physiologically-tolerated derivates such as salts, esters, N-oxides, and the like are mixed with additives customary for this purpose, such as vehicles, stabilizers, or inert diluents, and converted by customary methods into suitable forms for administration, such as tablets, coated tablets, hard or soft gelatin capsules, aqueous, alcoholic or oily solutions.

Examples of suitable inert vehicles are conventional tablet bases such as lactose, sucrose, or cornstarch in combination with binders such as acacia, cornstarch, gelatin, with disintegrating agents such as cornstarch, potato starch, alginic acid, or with a lubricant such as stearic acid or magnesium stearate.

Examples of suitable oily vehicles or solvents are vegetable or animal oils such as sunflower oil or fish-liver oil. Compositions can be effected both as dry and as wet granules. For parenteral administration (subcutaneous, intravenous, intraarterial, or intramuscular injection), the chemical entity or its physiologically tolerated derivatives such as salts, esters, N-oxides, and the like are converted into a solution, suspension, or expulsion, if desired with the substances customary and suitable for this purpose, for example, solubilizers or other auxiliaries.

Examples are sterile liquids such as water and oils, with or without the addition of a surfactant and other pharmaceutically acceptable adjuvants. Illustrative oils are those of petroleum, animal, vegetable, or synthetic origin, for example, peanut oil, soybean oil or mineral oil. In general, water, saline, aqueous dextrose and related sugar solutions, and glycols such as propylene glycols or polyethylene glycol are preferred liquid carriers, particularly for injectable solutions.

The preparation of compositions which contain an active component is well understood in the art. The active therapeutic ingredient is often mixed with excipients which are pharmaceutically acceptable and compatible with the active ingredient. Suitable excipients include, for example, water, saline, dextrose, glycerol, ethanol, or the like or any combination thereof. In addition, the composition can contain minor amounts of auxiliary substances such as wetting or emulsifying agents, pH buffering agents which enhance the effectiveness of the active ingredient.

An active component can be formulated into the composition as neutralized pharmaceutically acceptable salt forms. Pharmaceutically acceptable salts include the acid addition salts, which are formed with inorganic acids such as, for example, hydrochloric, or phosphoric acids, or such organic acids as acetic, tartaric, mandelic, and the like. Salts formed from the free carboxyl groups can also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

Salts of apoaequorin are preferably pharmaceutically acceptable salts. Other salts may, however, be useful in the preparation of the compositions according to the invention or of their pharmaceutically acceptable salts. Suitable pharmaceutically acceptable salts include acid addition salts which may, for example, be formed by mixing a solution of apoaequorin with a solution of a pharmaceutically acceptable acid such as hydrochloric acid, sulphuric acid, methanesulphonic acid, fumaric acid, maleic acid, succinic acid, acetic acid, benzoic acid, oxalic acid, citric acid, tartaric acid, carbonic acid or phosphoric acid.

Apoaequorin-containing compositions described herein are provided in the form of nutraceutical compositions where apoaequorin prevents the onset of or reduces or stabilizes various deleterious effects of neuronal inflammation. The term "nutraceutical" or "nutraceutical composition", for the purpose of this specification, refers to a food item, or a part of a food item, that offers medical health benefits, including prevention and/or treatment of disease. A nutraceutical composition according to the present invention may contain only apoaequorin as an active ingredient, or alternatively, may further comprise, in admixture with dietary supplements including vitamins, co-enzymes, minerals, herbs, amino acids and the like which supplement the diet by increasing the total intake of that substance.

Therefore, the present invention provides methods of providing nutraceutical benefits to a patient comprising the step of administering to the patient a nutraceutical composition containing apoaequorin. Such compositions generally include a "nutraceutically-acceptable carrier" which, as referred to herein, is any carrier suitable for oral delivery including aforementioned pharmaceutically-acceptable carriers suitable for the oral route. In certain embodiments, nutraceutical compositions according to the invention comprise dietary supplements which, defined on a functional basis, include immune boosting agents, anti-inflammatory agents, anti-oxidant agents, anti-viral agents, or mixtures thereof.

Immune boosters and/or anti-viral agents are useful for accelerating woundhealing and improved immune function; and they include extracts from the coneflowers, or herbs of the genus *Echinacea,* extracts from herbs of the genus *Sambuca,* and Goldenseal extracts. Herbs of the genus *Astragalus* are also effective immune boosters in either their natural or processed forms. *Astragalus* stimulates development of stem cells in the marrow and lymph tissue active immune cells. Zinc and its bioactive salts, such as zinc gluconate and zinc acetate, also act as immune boosters in the treatment of the common cold.

Antioxidants include the natural, sulfur-containing amino acid allicin, which acts to increase the level of antioxidant enzymes in the blood. Herbs or herbal extracts, such as garlic, which contain allicin, are also effective antioxidants. The catechins, and the extracts of herbs such as green tea containing catechins, are also effective antioxidants. Extracts of the genus *Astragalus* also show antioxidant activity. The bioflavonoids, such as quercetin, hesperidin, rutin, and mixtures thereof, are also effective as antioxidants. The primary beneficial role of the bioflavonoids may be in protecting vitamin C from oxidation in the body. This makes more vitamin C, or ascorbic acid, available for use by the body.

Bioflavonoids such as quercetin are also effective anti-inflammatory agents, and may be used as such in the inventive compositions. Anti-inflammatory herbal supplements and anti-inflammatory compounds derived from plants or herbs may also be used as anti-inflammatory agents in the inventive composition. These include bromolain, a proteolytic enzyme found in pineapple; teas and extracts of stinging nettle; turmeric, extracts of turmeric, or curcumin, a yellow pigment isolated from turmeric.

Another supplement which may be used in the present invention is ginger, derived from herbs of the genus *Zingiber.* This has been found to possess cardiotonic activity due to compounds such as gingerol and the related compound shogaol as well as providing benefits in the treatment of dizziness, and vestibular disorders. Ginger is also effective in the treatment of nausea and other stomach disorders.

Supplements which assist in rebuilding soft tissue structures, particularly in rebuilding cartilage, are useful in compositions for treating the pain of arthritis and other joint disorders. Glucosamine, glucosamine sulfate, chondroitin may be derived from a variety of sources such as Elk Velvet Antler. Marine lipid complexes, omega 3 fatty acid complexes, and fish oil are also known to be useful in treating pain associated with arthritis.

Supplements useful in treating migraine headaches include feverfew and *Gingko biloba.* The main active ingredient in feverfew is the sesquiterpene lactone parthenolide, which inhibits the secretions of prostaglandins which in turn cause pain through vasospastic activity in the blood vessels. Feverfew also exhibits anti-inflammatory properties. Fish oil, owing to its platelet-stabilizing and antivasospastic actions, may also be useful in treating migraine headaches. The herb *Gingko biloba* also assists in treatment of migraines by stabilizing arteries and improving blood circulation.

The invention will be more fully understood upon consideration of the following non-limiting Examples.

### EXAMPLES

Example 1. Pretreatment with Apoaequorin Protects Hippocampal CA1 Neurons from Oxygen-Glucose Deprivation. [Reference Example]

### Materials and Methods

### Subjects

Subjects were 142 adult male F344 rats (mean age 4.0 ± 0.1 mo.; Harlan). Subjects were maintained in an Association for Assessment and Accreditation of Laboratory Animal Care (AAALAC) accredited facility on a 14 hr light-10 hr dark cycle and housed individually with free access to food and water.

### Surgery

Rats were given ibuprofen water (15 mg/kg/day) for at least one day before and two days after surgery. On the day of surgery, rats were anesthetized with isoflurane and mounted on a stereotaxic apparatus. Under aseptic conditions, bilateral 26-gauge stainless steel guide cannulae were implanted in the dorsal hippocampus (relative to bregma: AP -3.5 mm, L ± 2.6 mm, V -3.0 mm). Cannulae were secured to the skull with stainless steel screws and acrylic cement. Stainless steel caps were placed in the guide cannulae to prevent occlusion, and rats were allowed to recover at least 7 days prior to infusion.

### Intrahippocampal Infusions

The aequorin protein is made up of two components, apoaequorin and coelenterazine. The apoaequorin component (AQ) contains the EF-hands that bind Ca²⁺ [51] and thus was the component used in the current studies. Rats were given an infusion of AQ in zero Ca²⁺ artificial cerebral spinal fluid (aCSF; in mM: 124.00 NaCl, 2.80 KCl, 2.00 MgSO₄, 1.25 NaH₂PO₄, 26.00 NaHC03, 10.00 D-glucose, and 0.40 Na-ascorbate), which also contained 6% DMSO to facilitate neuronal uptake of AQ. Rats received bilateral infusions (0.5 µl/hemisphere) over 60s, and the infusion cannulae remained in place for an additional 2 min to ensure diffusion away from the tip. The 33-gauge infusion cannulae were cut to extend 0.5 mm beyond the guide cannulae. To determine the dosage-dependent neuroprotection of AQ, animals were infused with 0.4, 1, or 4% AQ (w/v; Quincy Bioscience, Madison, WI) in one hemisphere (counterbalanced), and the other was infused with vehicle. In addition, a subset of rats was infused with vehicle (0% AQ) in both hemispheres to serve as a control (n = 11 for each group).

### Slice Preparation

To determine the neuroprotective effect of AQ on an acute brain slice model of ischemia, 94 male F344 rats were used (mean age 4.4 ± 0.2 mo.). Brain slices were prepared as previously described from control rats (0% AQ, n = 10) or from rats infused with AQ at one of the following time points after infusion: 1 hr (n = 10), 1 day (n = 10), 2 days (n = 10), 3 days (n = 10), or 5 days (n = 5). Briefly, rats were deeply anesthetized with isoflurane, perfused through the ascending aorta with ice-cold, oxygenated (95% O₂ / 5% CO₂) sucrose-CSF (in mM: 206.00 sucrose, 2.80 KCl, 2.00 MgSO₄, 1.25 NaH₂PO₄, 1.00 CaCl₂, 1.00 MgCh, 26.00 NaHCO₃, 10.00 D-glucose, and 0.40 Na-ascorbate) and the brain rapidly removed and placed in ice-cold, oxygenated sucrose-CSF. The brain was blocked near the site of the cannula, and 400 µm thick coronal slices were cut on a temperature-controlled Vibratome as described previously. Only the first 5 slices immediately posterior to the cannula placement (and devoid of any visible cannula track) were collected and used in the experiments described below. Slices were incubated on a mesh net submerged in oxygenated (95% O₂ / 5% CO₂), aCSF (composition in mM: 124.00 NaCl, 2.80 KCl, 2.00 MgSO₄, 1.25 NaH₂PO₄, 2.00 CaCl₂, 26.00 NaHCO₃, 10.00 D-glucose, and 0.40 Na-ascorbate) at 35 °C. Following a 1 hr recovery, slices were subjected to 5-min oxygen-glucose deprivation (OGD) to induce ischemia. OGD was induced by transferring the slices to a 35 °C solution of fructose-CSF (in which an equimolar concentration of fructose was substituted for glucose), which was bubbled with 95% N₂ / 5% CO₂ (in which N₂ replaced O₂). Following the OGD, slices were transferred to a 35 °C solution containing oxygenated aCSF plus 0.2% trypan blue (Sigma-Aldrich, St. Louis, MO) for 30 min reperfusion. Trypan blue penetrates dead and dying cells and stains them blue while leaving living cells unstained. The slices were then briefly rinsed in room temperature, oxygenated aCSF and immediately fixed in 10% neutral buffered formalin overnight in the refrigerator. Slices were cryoprotected with 30% sucrose for a minimum of 1 day, after which they were subsectioned on a cryostat at 40 µm, mounted onto gelatin-coated slides, dehydrated in increasing steps of alcohol, and coverslipped with Permount.

### Cell Counts

The slices were examined under an upright microscope (Olympus BX51) equipped with a digital camera (Olympus DP70) and a 10X objective. Within each 40-µm subsection, a photograph was taken of the CA1 cell body layer (at the tip of the upper blade of the dentate gyrus). To avoid excessive staining due to neuronal damage as a result of the initial hippocampal slice preparation, only interior subsections were photographed for analysis. An individual blind to treatment condition then counted the number of trypan blue stained neurons located throughout the entire image. Data were counted from only one subsection. Percent neuroprotection was assessed for each animal by normalizing the data from the AQ-treated hemisphere to the vehicle-treated hemisphere.

### Western Blot Analysis

To determine how long AQ remained in the dorsal hippocampus following an infusion, 24 adult male F344 rats (mean age 4.2 ± 0.1 mo.) were infused with 4% AQ in both hemispheres. Rats were anesthetized with an overdose of isoflurane at 1 h (n = 4), 1 d (n = 7), 2 d (n = 7), or 3 d (n = 6) after infusion, and the brain was removed, rapidly frozen on dry ice, and stored at -80 °C. From each rat, two bilateral brain regions (dorsal hippocampus and ventral hippocampus; dhpc and vhpc, respectively) were dissected out and homogenized separately. Samples were centrifuged at 4000 rpm, and the supernatant removed and measured using a Bradford protein assay kit (Bio-Rad, Hercules, CA). Protein samples were normalized (50 or 150 µg/lane) and loaded for SDS-PAGE (10%). Proteins were transferred onto PVDF membranes using a semidry transfer apparatus (Bio-Rad, Hercules, CA). Membranes were then incubated for 2 hours in blocking buffer (3% nonfat dry milk) after which they were incubated in primary antibody (overnight at 4 °C; 1:5000 mouse anti-aequorin [Millipore, Billerica, MA] or 1:1000 rabbit anti-β -actin [Cell Signaling Technology, Boston, MA]) followed by secondary antibody (90 min; 1:5000 goat anti-mouse [Santa Cruz Biotechnology, Santa Cruz, CA] or 1:5000 goat anti-rabbit [Millipore]). Membranes were then washed (0.05% Tween-20 in tris-buffered saline), placed in a chemiluminescence solution (Santa Cruz Biotechnology), and exposed to autoradiographic film (Hyperfilm MP). Images were taken and densitometry was performed using NIH Image J Software. A band was considered positive if the optical density value of the band (minus the background for each lane) was greater than 2 standard deviations above the mean of the ventral hippocampus bands. From this quantification, a positive band was observed in 100% of the 1 hour bands, 83% of the 1 day bands, 29% of the 2 day bands, 0% of the 3 day bands, and 0% of the vhpc lanes. Comparison was made to the ventral hippocampus because this is an adjacent brain structure that should not contain AQ, and was thus used as a negative control structure.

### Quantitative RT-PCR

Twelve male rats (each at 3.8 mo.) received unilateral infusions of 4% AQ as described above, and tissue was collected at 1 hour, 1 day, or 2 days post-infusion (n = 4 per group). Hippocampi were excised and immediately placed into TRIzol reagent (Life Technologies Corp., Carlsbad, California). Tissues were homogenized using a 25-gauge needle and syringe, and samples were stored at -80 °C until RNA isolation. RNA isolation from all tissues was performed at the same time using the TRIzol method (Life Technologies Corp, Carlsbad, CA), according to manufacturer's instructions. Isolated RNA was dissolved in 50 µl RNase free H₂O, and RNA purity was calculated based on the absorbance ratio of 260 nm and 280 nm. An absorbance reading between 1.8 and 2.1 was considered sufficiently pure to proceed with reverse transcription. Samples presenting with a ratio less than 1.8 were further purified utilizing the Qiagen RNeasy MinElute Cleanup Kit (Qiagen, Valencia, CA) according to manufacturer's instructions, and purified RNA was resuspended in 50 µl of RNase free H₂O. Total RNA from all samples was reverse transcribed to cDNA using the Qiagen RT2 HT First Strand Kit-96 (Qiagen). Samples were amplified in triplicate in 96-well plates utilizing primers specific for rat IL-10 and β-actin (RT2 qPRC Primer Assay; Qiagen) and RT² SYBR Green qPCR mastermix (Qiagen) on a StepOne Real Time PCR system and software (Life Technologies Corp., Carlsbad, California). Changes in IL-10 gene expression with AQ treatment relative to vehicle treatment were calculated using the Pfaffl equation, normalizing to β-actin expression in corresponding samples at each time-point and compared to vehicle-treated hippocampi isolated from each rat. Primer efficiency was calculated based on dilution curves of two randomly selected samples for IL-10 and β-actin. β-Actin expression was not altered by infusion of AQ when compared to tissue infused with aCSF, indicating AQ infusion did not generally or nonspecifically affect gene transcription.

### Gene expression arrays

cDNA was taken from the rats used for RT-PCR (see Methods). PCR analyses focused on overall genetic markers of inflammatory cytokines and receptors, with Qiagen's RT2 Profiler Arrays conducted as per manufacturer protocol. Briefly, 2X RT2 SYBR Green Mastermix, cDNA (see above), and RNase-free water were combined, and 25 µl of this mix was added to each well of the 96-well PCR Profiler Array well plate. Samples were run using StepOne Real Time PCR system and software, and those samples with multiple melt curves were eliminated from analysis (n = 2 excluded). One animal from the study had to be eliminated altogether, due to general variability in gene expression over two standard deviations from the mean. Gene expression changes were calculated using Qiagen's Web-Based RT2 Profiler PCR Array Analysis Software v3.5. Data analysis and statistics

Statistical analyses were performed using Statview (v 5.0; SAS Institute, Inc., Cary, NC). An ANOVA was used to evaluate treatment effects. Fisher's PLSD was used for *post hoc* comparisons. Data are reported as the mean ± standard error of the mean.

### Results

### Oxygen-Glucose Deprivation Results in Significant Cell Death

Acute hippocampal slices were prepared, exposed to 5 min oxygen-glucose deprivation (OGD), and stained by transferring them to an oxygenated-aCSF that contained trypan blue (see methods). As can be seen in Figure 1, OGD resulted in significantly more cell death compared with control slices that did not undergo OGD. An ANOVA analyzing the average number of trypan blue-stained cells in ischemic or nonischemic conditions demonstrated a statistically significant effect of ischemia, *F*(1, 12) = 9.65, *p* < .01. These findings are consistent with prior studies indicating that OGD results in significant cell death in CA1 region of the hippocampus [52].

### Decreased Cell Death with Apoaequorin Treatment

To examine the potential neuroprotective effects of an intra-hippocampal infusion of apoaequorin (AQ) prior to OGD, rats were infused with 0, 0.4, 1, or 4% AQ 24 hr prior to OGD (see Figure 2A). AQ was neuroprotective in a dose-dependent manner such that intra-hippocampal infusions with either 1% or 4% AQ prior to ischemia resulted in a significant increase in neuroprotection compared to vehicle (0% AQ) infusion, F(3, 40) = 3.61, *p <* .05 (Figure 2B&C). *Post hoc* analysis revealed that infusions of 1 or 4% AQ significantly enhanced neuroprotection relative to the 0% AQ group, *p* < .01, and that infusion of 0.4% AQ was not statistically different from any of the other groups. It was also worth noting that that amount of neuroprotection was not different between the 1% and 4% AQ treatment groups.

To evaluate the time course over which AQ is neuroprotective, rats were infused with 4% AQ at various times (1 h, 1 d, 2 d, 3 d, or 5 d) prior to OGD (Figure 3A). One-way ANOVA indicated there was a significant effect of time on the ability of an intra-hippocampal infusion of AQ to protect neurons from a subsequent OGD, *F*(5, 49) = 3.35, *p* < .05. Post-hoc tests revealed that the neuroprotective effects of AQ required at least 1 day to emerge and that they lasted at least 2 days (*p* < .05 for each time point). No statistically significant neuroprotection was observed when slices were subjected to OGD 3 or 5 days following AQ infusion (*p =* .10 and *p* = .47, respectively).

### Western Blot Analysis of Apoaequorin

To determine how long AQ remains within the dorsal hippocampus following an intra-hippocampal infusion, AQ protein levels were measured using Western blot analysis at different times (1 h, 1 d, 2 d, or 3 d) following bilateral infusion of 4% AQ into the dorsal hippocampus. Figure 3C illustrates that within dorsal hippocampus AQ is present at 1 h and 1 d, barely visible at 2 d and no longer present by 3 d post-infusion. Thus, positive bands were observed in 100% of the 1 h, 83% of the 1 d, 29% of the 2 d, and 0% of the 3d lanes. As expected, AQ was not detected in the ventral hippocampus (vhpc), which was used as a negative control structure given its distance from the injection site (see Figure 3C). To ensure that enough protein was loaded into the gels to enable visualization of extremely faint bands, Western blots were repeated in a subset of animals, but the gels were loaded with 150 µg of protein per lane (instead of the normal 50 µg per lane). In these blots, additional bands came through in the 2- and 3-day lanes such that 57% of the 2 d and 25% of the 3 d lanes had positive bands suggesting that AQ is detectable within dorsal hippocampus for up to 3 days following dorsal hippocampal infusions. Importantly, no time-dependent changes were observed when samples were stained for β-actin, suggesting that these differences reflected time-dependent changes in the presence of AQ and not a general change in protein content (see Figure 3C).

### Cytokine and Chemokine Expression Following AQ-infusion

That an intra-hippocampal infusion of AQ resulted in significant neuroprotection at time points when very little protein was present suggests that AQ may trigger some cascade of events that ultimately protect neurons from an ischemic insult. One possibility is that AQ induces a pre-conditioning-like effect, resulting in reduced cell death at later time points. Ischemic pre-conditioning is a phenomenon whereby a brief ischemic episode attenuates damage caused by a subsequent more severe ischemic insult. Recent evidence has shown that multiple cytokines and chemokines are associated with ischemic preconditioning. Given the link between ischemic pre-conditioning and alterations in cytokine production, we tested the hypothesis that an infusion of AQ may lead to an increase in cytokine or chemokine expression, which may ultimately impact the ability of neurons to tolerate a later ischemic insult. RT-PCR was used to investigate mRNA changes in the anti-inflammatory cytokine interleukin-10 (IL-10), and PCR arrays were used to look at multiple gene expression changes following AQ infusion. Adult rats received an infusion of 4% AQ in one hemisphere and vehicle in the other as described (see Methods). At different times following the AQ infusion (1 h, 1 d, or 2 d later), the hippocampi were removed and quantitative RT-PCR was performed to evaluate time- and treatment-dependent changes. One-way ANOVA indicated a significant difference between the four treatment groups, F(3, 19) = 9.55, *p <* .0005. *Post hoc* analyses revealed that IL-10 mRNA was significantly increased 1 h after infusion in the AQ- relative to the vehicle-treated hemisphere (*p* < .001; see Figure 4A). Moreover, this AQ-induced enhancement of IL-10 expression at 1 h was significantly larger than the enhancement at 1 d (*p* < .001) or 2 d (*p* < .001). Although IL-10 expression was increased 2-3 fold at the later time points, these were not statistically significantly different from vehicle-treated hemispheres, suggesting that the significant increase in IL-10 observed at 1 hour may be due to an acute response to AQ infusion.

To investigate whether the AQ-related change in cytokine expression was restricted to IL-10 rather than being part of a more global change in mRNA expression patterns, PCR arrays were performed. Eighty-two total genes related to cytokine and chemokine responses were surveyed. Among these, 80 genes were present to varying extents in the control hemisphere and 2 genes (CCR8, chemokine receptor 8; and CRP, C-reactive protein) were not detected. Of the 80 genes that were detectable, only 16 were significantly different between AQ- and vehicle-treated hemispheres (see Table 1, data organized by response time). The majority of genes were increased at 1-hour post-AQ infusion, and thereafter decreased to or near baseline levels by 1 day. Of the 8 that were significantly upregulated at 1 hour, only one remained elevated through the 2-day post-infusion time point, Chemokine ligand 10 (CXCL10). Six genes were not significantly upregulated at 1 hour but were upregulated at 1-day post-AQ infusion. Of these six, only two did not remain elevated at 2 days - Chemokine ligand 11 (CXCL11) and Interleukin-1 receptor type II (IL-1rII). Only two genes were significantly upregulated exclusively at the 2 days post-AQ infusion time point - Chemokine receptor 1 (XCR1) and Complement component 3 (C3). These results indicate that an infusion of AQ into the dorsal hippocampus has a dramatic effect on cytokine and chemokine mRNA expression at both short- and long-term time points.

### Discussion

The current study demonstrates that the calcium binding protein apoaequorin (AQ) is neuroprotective in a time- and dose-dependent manner when administered prior to ischemic injury. Intra-hippocampal infusion of either 1% or 4% AQ resulted in significantly fewer dead or dying neurons as compared to animals infused with control (see Figure 2). This neuroprotection was time-dependent, in that it took up to 1 or 2 days to develop and it subsided by 3 to 5 days. Neuroprotection may involve a preconditioning like effect, whereby an AQ infusion modulates cytokine and chemokine expression, which subsequently protects neurons from oxygen-glucose deprivation (OGD).

Previous studies have suggested a neuroprotective role for CaBPs. For example, neurons that contain the CaBP calbindin are more resistant to excitotoxic and ischemia-related injuries than neurons that lack calbindin. In addition, some studies have noted that calbindin expression increases following traumatic brain injury and ischemia, indicating that calbindin may be increased to maintain Ca²⁺ homeostasis and protect against excitotoxicity. Likewise, using either gene therapy or protein transduction, overexpression of CaBPs prior to ischemia has also been found to be neuroprotective. In contrast, that calbindin is present in both the dentate gyrus (an area resistant to ischemic cell death) and CA1 (an area vulnerable to cell death) has been used as an argument against a role for calbindin in neuroprotection. Finally, others have reported that recovery from ischemia is enhanced in calbindin knockout mice. Since these were not inducible knockouts, it is possible that other compensatory mechanisms played a role in the observed neuroprotection.

Studies examining the effect of artificial calcium chelators (e.g., BAPTA-AM, EGTA, etc...) on excitotoxicity have had mixed results, with some studies finding neuroprotection and others finding enhanced vulnerability to cell death. Nikonenko et al. demonstrated neuroprotection in rat organotypic hippocampal slice cultures following OGD in slices treated with EGTA, BAPTA, Mibefradil, Kurtoxin, Nickel, Zinc, and Pimozide. On the contrary, Abdel-Hamid and Baimbridge loaded cultured hippocampal neurons with the calcium chelator BAPTA-AM and found enhanced glutamate excitotoxicity in those neurons. The authors' suggest that the presence of artificial calcium chelators interferes with normal Ca²⁺-dependent mechanisms that prevent Ca²⁺ influx into the cell. These opposing results may be due to a number of factors, including the mode of inducing excitotoxicity, the type of Ca²⁺ chelator used, or the use of cultured neurons as compared to acute brain slices.

Interestingly, when the AQ protein was most readily detected in the dorsal hippocampus, at 1-hour post-infusion, neuroprotection was not observed (see Figure 3). Although it is unknown how or whether AQ enters the cell, the current study used DMSO with AQ for infusions, which is used to transport drugs across membranes. Thus, it is likely that AQ had the opportunity to enter cells. Moreover, the centrifugation process for the Western blot samples was designed to isolate intracellular components of the cell (by centrifuging at a low speed), and AQ's presence in these samples strongly suggests its presence within the cells. Although significant neuroprotection was evident at 1 and 2 days post-infusion, much less AQ was evident in dorsal hippocampus (Figure 3C), suggesting that neuroprotection did not merely result from immediate effect of AQ binding Ca²⁺. Rather, the data suggest that neuroprotection results from a cascade of events caused by the AQ infusion. Since the neuroprotective effects were observed at 1 and 2 days post-infusion when the protein was barely present or not detected (but not at 1 hour when AQ expression was at its highest), this cascade is likely to be due to other AQtriggered mechanisms, including a pre-conditioning-like effect post-infusion. This type of an effect would take time to develop, and would explain why neuroprotection was not immediately observed (e.g., 1 hr post-infusion). Preconditioning may also explain why robust neuroprotection was observed at 1 or 2 days post-infusion, despite lower detection of the protein at these time points. While the exact mechanisms are currently unknown, studies have implicated cytokines and chemokines in preconditioning.

To investigate whether the observed neuroprotection following AQ infusion is due to a preconditioning-like effect, we measured changes in IL-10 mRNA, an anti-inflammatory cytokine known to be involved in preconditioning. A statistically significant increase in IL-10 mRNA was observed 1 hour after infusion. Although not statistically significant, a biologically significant (>2-fold) increase in IL-10 mRNA continued to be observed for up to 2 days following AQ infusion (see Figure 4A). Anti-inflammatory cytokines can act by recruiting cell populations that are protective through cytokine secretion, which in turn prevent or down-regulation the induction of a damaging pro-inflammatory immune response, actively protecting against future insult. The increased IL-10 expression at 1-hour post-AQ infusion may be serving as a protective primer for the upcoming OGD insult such that 1-2 days later, the brain is fully primed and better able to withstand an ischemic insult. This effect is short-lived such that by 3-5 days post-AQ infusion, little to no neuroprotection is evident.

Given that an increase in IL-10 mRNA at 1 hour post-AQ infusion suggests a preconditioning-like effect, multi-gene PCR arrays were used to evaluate the effects of AQ on the expression of a wide variety of cytokines and chemokines (see Table 1). These studies revealed that AQ infusion differentially regulates, in a time-dependent manner, expression of a number of cytokines and cytokine receptor genes compared to the vehicle-treated hemisphere. Of the 82 total genes examined in the array, 16 were significantly upregulated following infusion of AQ. Within these 16, a time-dependent effect was evident, such that 8 were rapidly upregulated immediately following AQ infusion whereas the remaining 8 were upregulated only after a 1- or 2-day delay.

**Table 1. Fold change in genes following 4% AQ infusion, grouped by response time**

| | **Time From AQ Infusion** | | |
|---|---|---|---|
| **Fast Responders (within 1 hour)** | **1 Hour** | **1 Day** | **2 Days** |
| Chemokine ligand 1 (CXCL1) | 19.36† | 1.77 | -1.81 |
| Chemokine ligand 3 (CCL3) | 20.07† | 8.85^{∗} | 1.15 |
| Chemokine ligand 4 (CCL4) | 33.89† | 6.20^{∗} | 1.68 |
| Chemokine ligand 10 (CXCL10) | 9.59^{∗} | 6.27 | 8.45^{∗} |
| Interleukin 1 alpha (IL-1α) | 36.63† | 1.23 | 1.25 |
| Interleukin 1 beta (IL-1β) | 32.46† | 8.94^{∗} | 1.43 |
| Interleukin-10 (IL-10) | 5.26^{∗} | 4.27 | 3.14 |
| Tumor necrosis factor (TNF-α) | 23.15† | 4.64 | -1.29 |

| **Slower Responders (within 1 day)** | | | |
|---|---|---|---|
| CD40 ligand | 1.62 | 17.91† | 7.15^{∗} |
| Chemokine ligand 9 (CXCL9) | 1.27 | 26.46† | 13.47^{∗} |
| Chemokine ligand 11 (CXCL11) | 4.47 | 15.22† | 3.84 |
| Chemokine receptor 3 (CXCR3) | 1.17 | 35.51† | 11.66 † |
| Interleukin 1 receptor, type II (IL-1rII) | 2.09 | 6.90^{∗} | -1.16 |
| Interleukin 2 receptor, beta (IL-2rβ) | 1.74 | 11.92† | 6.70^{∗} |

| **Slowest Responders (within 2 days)** | | | |
|---|---|---|---|
| Chemokine receptor 1 (XCR1) | -2.04 | 3.19 | 8.81^{∗} |
| Complement component 3 (C3) | 2.07 | 3.93 | 10.11† |

Numbers represent fold change from vehicle-infused hemisphere (^{∗} *p <* .05; † *p* < .01).

Of the cytokines that were upregulated post-AQ infusion, effects of preconditioning have been examined in only four: (1) interleukin-1β (IL-1β), (2) IL-10, (3) tumor necrosis factor-α (TNF-α), and (4) complement component 3 (C3). All four of these cytokines have been shown to be increased following preconditioning. IL-1β, a pro-inflammatory cytokine, has been shown to increase within 6 hours after preconditioning after which it returns to baseline within 3-4 days. This is consistent with the present study, which demonstrates a rapid increase in IL-1β mRNA followed by a return to baseline levels by 2 days post-AQ (Table 1). While IL-1β is a pro-inflammatory cytokine, moderate increases can be neuroprotective. Likewise, IL-10 has also been shown to rapidly increase following preconditioning, with a fairly quick return to baseline. Here we show using both quantitative RT-PCR (Figure 4) and PCR arrays (Table 1) that IL-10 is significantly upregulated at 1 hr post-AQ infusion. IL-10 has been shown to decrease the release of TNF-α and reduce brain injury following focal ischemia in rats. Following preconditioning, TNF-α is rapidly upregulated, persists for up to 2 days, and is no longer detected after 3-4 days. The current experiments demonstrate an increase in TNF-α gene expression at 1 hour, but not at 1 or 2 days post-AQ infusion. C3 was significantly upregulated 24 hours following lipopolysaccharide (LPS) preconditioning. Here, a significant increase in C3 gene expression was observed at 2 days after AQ-infusion. Activation of the complement host defense system, including C3, has been shown to have both damaging and protective effects. Taken together, these data indicate that the increase in IL-1β, IL-10, TNF-α, and C3 in the current experiment may be one reason for the neuroprotective effects of AQ-infusion.

While only four of the upregulated cytokines have been examined in preconditioning, almost all of the 16 have been examined following cerebral ischemia. Only chemokine ligand-9 (CXCL9), chemokine ligand-11 (CXCL11), and chemokine receptor-1 (XCR1) have not been, to our knowledge, previously examined with their relations to cerebral ischemia. Of the other cytokines, all have been shown to increase following ischemia, except Interleukin-2 receptor, beta (IL-2rβ). Under normal conditions, IL-2rβ is found within the cell membrane of hippocampal CA1 pyramidal neurons. Following ischemia, IL-2rβ not only decreases within CA1, but it also translocates from the cell membrane to the cytoplasm and nucleus. How some cytokines function following ischemia likely depends upon their expression patterns, which may influence when and whether they are neuroprotective or not. For example, CD40 ligand plays a role in inflammation and tissue injury, and it is upregulated following focal ischemia. However, CD40 ligand also protects neurons from neuronal stress and deficiency in CD40 ligand results in neuronal dysfunction, indicating that CD40 ligand is important for general neuronal function. The present data indicate a significant increase in CD40 ligand at both 1 and 2 days post-AQ infusion. This sustained increase in CD40 ligand may contribute to the time course of our observed neuroprotection. Although beyond the scope of the present study, it will be important (and the data suggest worthwhile) to further assess the neuroprotective effects of AQ over a longer time frame using an *in vivo* model of ischemia.

In conclusion, the current experiments support the hypothesis that AQ protects neurons against ischemia when administered directly to the brain prior to an ischemic insult. These effects are both dose- and time-dependent such that a single intrahippocampal infusion of AQ protects neurons from OGD for up to 2 days. Moreover, AQ infusions activated cytokine and chemokine gene expression in a manner similar to those seen with ischemic preconditioning. Thus, pretreatment with AQ may be an effective way to protect neurons against ischemic stroke by acting as a chemical preconditioning agent.

Example 2. Effect of intrahippocampal infusion of Apoaequorin on cytokine protein expression. [Reference Example]

In previous experiments, our lab has shown that a single intrahippocampal infusion of AQ 24 and 48 hours prior *in vitro* ischemic insult significantly reduces cell death (Detert et al., 2013). It has also been found that there are concurrent changes in cytokine mRNA after AQ infusion, including interleukin-10 (IL-10) and tumor necrosis factor-alpha (TNF-α; Detert et al., 2013). These data indicate that AQ's neuroprotective mechanism may involve modulation of certain anti- and pro-inflammatory molecules, possibly involving a preconditioning-like effect. The current study was designed to further investigate whether cytokine protein expression also changes in a time-dependent manner after an intrahippocampal infusion of AQ. By focusing on possible changes in protein levels, we hope to gain a better understanding of the extent to which AQ modulates various cytokines and ultimately understand the mechanism by which AQ protects neurons from oxygen-glucose deprivation.

Our lab has previously shown that an infusion of apoaequorin (AQ) into the CA1 region of hippocampus is neuroprotective in a time- and dose-dependent manner (Detert et al., 2013).

Significant neuroprotection was observed at 1 and 2 days, but not 1 hour after AQ was infused. This was paralleled by altered cytokine mRNA expression, suggesting that this ischemic neuroprotection may involve a neuroimmunomodulatory response (Detert et al., 2013).

Induction of a mild stress stimulus can trigger ischemic preconditioning via modulation of inflammatory cytokine expression (Gidday, 2006).

IL-10 protects neurons from ischemic damage both *in vitro* and *in vivo* (Grilli et al., 2000).

IL-10 inhibits the upregulation of TNF-α, a proinflamatory cytokine, which is involved in the pathologic mechanisms of hemorrhagic stroke (Ewen et al., 2013).

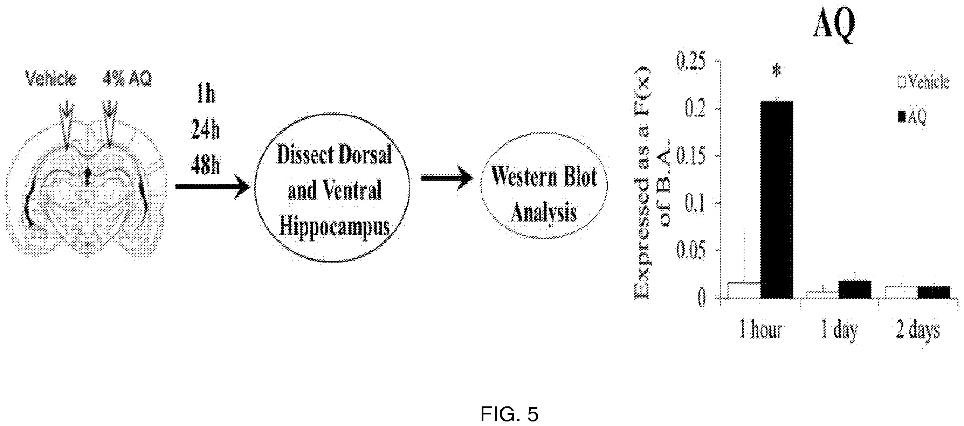

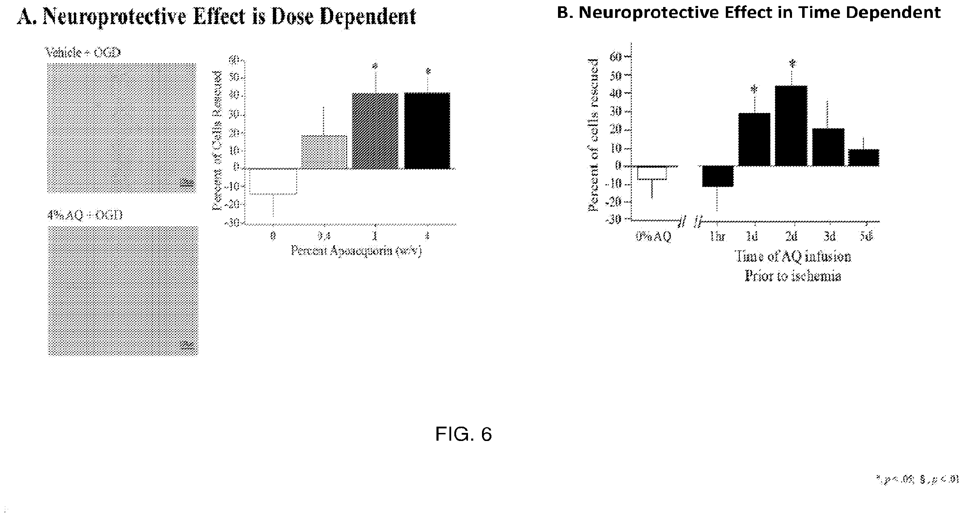

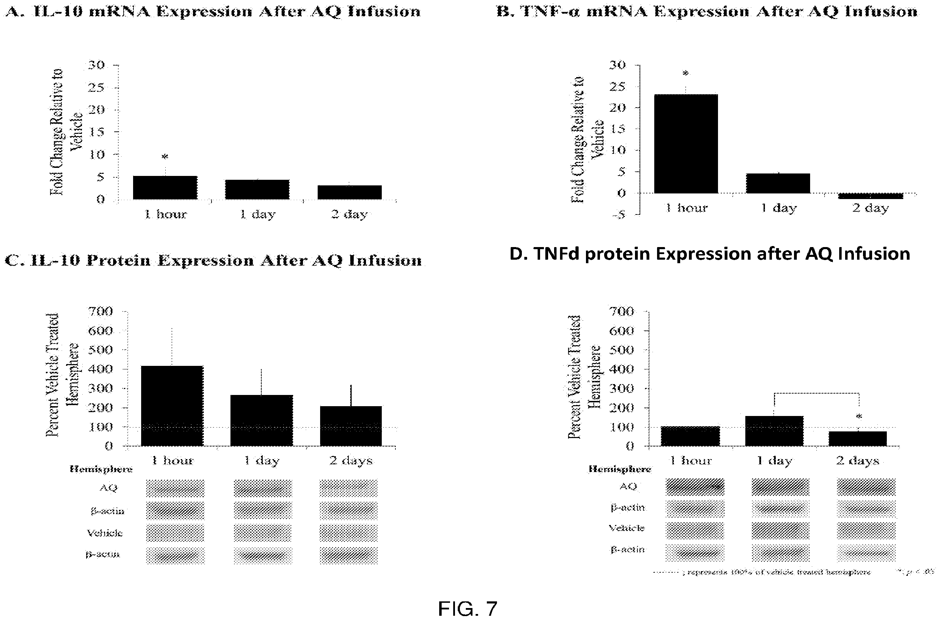

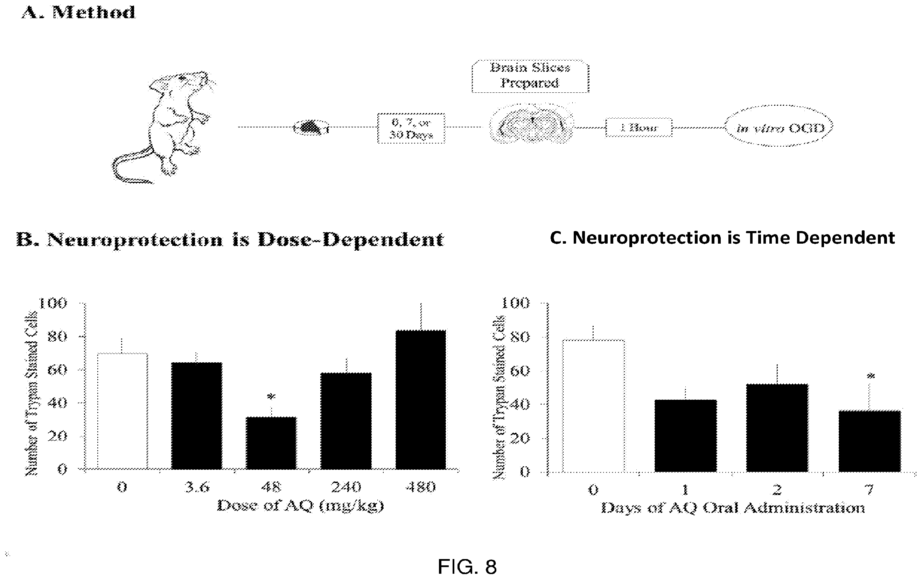

The present example demonstrates intrahippocampal infusion of AQ initiates a neuroimmunomodulatory response that triggers changes in IL-10 and TNF-α protein expression. Data supporting this conclusion is provided in Figs. 5, 6A-B, 7A-D and 8A-C.

Example 3. The Neurotherapeutic Effects of the Calcium Binding Protein Apoaequorin.

Calcium-binding proteins (CaBPs) mitigate ischemic cell death.

Data from our lab show that the CaBP apoaequorin (AQ) is neuroprotective when infused into the dorsal hippocampus prior to *in vitro* ischemia, and leads to a time-specific elevation of IL-10 and TNF-α mRNA, suggesting a role for AQ in preconditioning (Detert et al., 2013).

The present example demonstrates that single hippocampal infusion of AQ will differentially modulate IL-10 and TNF-α protein expression.

Calcium toxicity is evident in normal aging. According to the calcium hypothesis of aging, dysregulation of calcium homeostasis contributes to cognitive decline in normal aging (Khachaturian, 1987).

There is an age-related reduction in CaBPs (DeJong et al., 1996; Bu et al., 2003; Moyer et al., 2011), and findings from our lab demonstrate reduced CaBP expression in the hippocampus, a structure important for trace fear learning (McEchron et al., 1998). Trace fear conditioning is impaired in normal aging (Villarreal et al., 2004; McEchron et al., 2004; Moyer et al., 2006). Mitigating excess calcium leads to improved cognitive function in aging animals (Deyo et al., 1989; Veng et al., 2003).

This example further demonstrates that single hippocampal infusion of AQ will mitigate aging-related deficits in acquisition of trace fear conditioning.

Oral administration was used as a delivery method. Using the hazelnut spread Nutella^{®} or peanut butter as a vehicle, delivery of compounds to rats can be accomplished orally (Isaksson et al., 2011; Cundell et al., 2003).

Recent data from our lab demonstrate AQ is neuroprotective when administered orally at a single dose prior to *in vitro* ischemia (Adams et al., SfN 2013). This example further demonstrates neuroprotective effects of AQ oral administration are dose- and time-dependent.

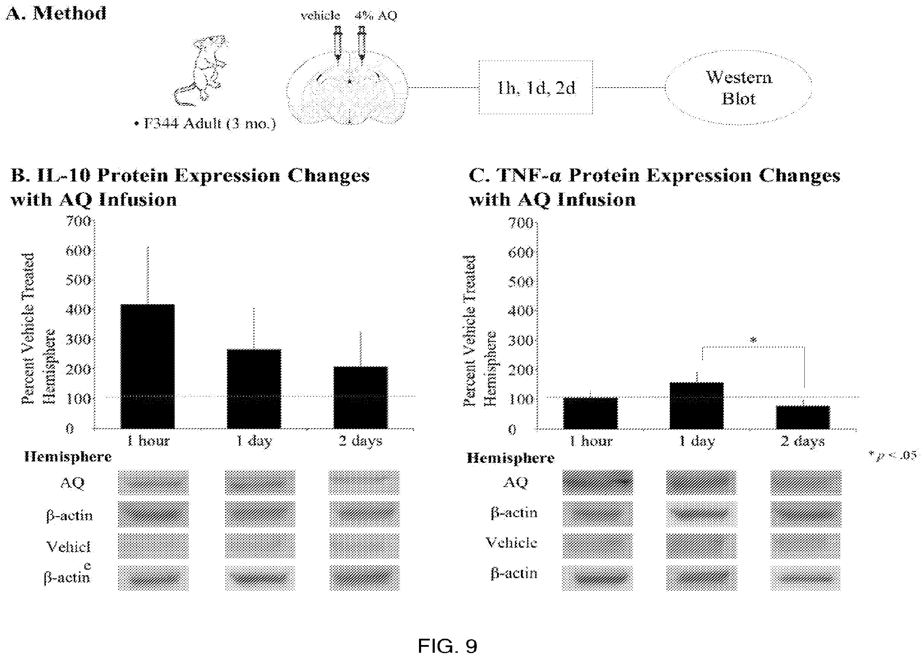

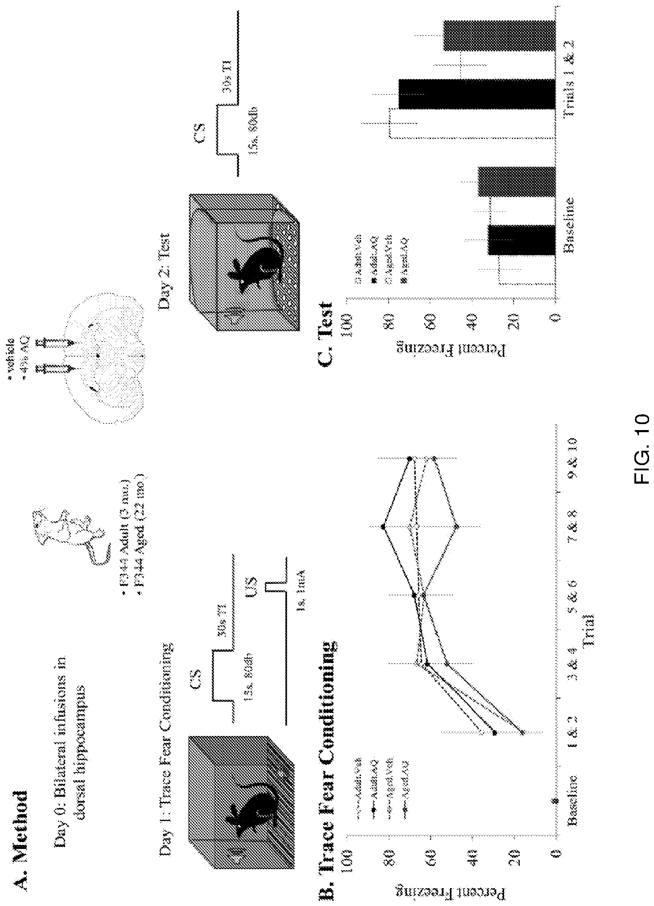

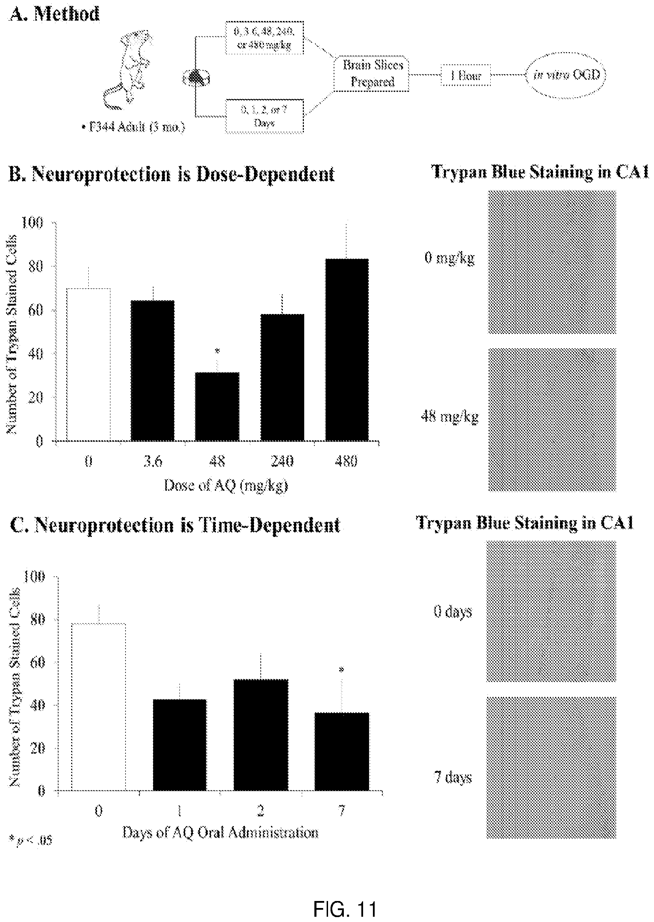

Figs. 9A-C, 10A-C and 11A-C support the following conclusions. Direct infusion of AQ results in altered IL-10 and TNF-α protein expression relative to vehicle. Both IL-10 and TNF-α show differential expression patterns following AQ infusion, indicating AQ's neuroprotective effects may be mediated by an immunomodulatory response.

AQ infusion did not rescue trace fear learning impairments in aging animals, and it did not interfere with learning of this task in adults. Aging rats demonstrate decreased freezing to the tone 24 h following conditioning relative to adults, but AQ administration did not lead to increased freezing in aging rats as was predicted.

Oral administration of AQ results in neuroprotection that is time- and dose-dependent. A dose of 48 mg/kg of AQ, and 7 days of oral administration led to a significant reduction in cell death following ischemia.

Example 4. Oral administration of AQ is neuroprotective in an acute slice model.

Our lab has recently demonstrated that apoaequorin (AQ) is neuroprotective in an acute brain slice model of ischemic stroke called oxygen glucose deprivation (OGD). Rats that received a 4% AQ infusion demonstrated decreased cell death following OGD (Detert et al., 2013).

This example demonstrates decrease in cell death is due to an immunomodulatory mechanism, involving time-dependent changes in cytokine mRNA.

Oral administration of compounds to rats was accomplished by using the hazelnut spread Nutella^{®} (Isaksson et al., 2011) or peanut butter (Cundell, et al., 2003) as a vehicle. Recently, AQ has been shown to be non-toxic when administered via gavage to rats (Moran, et al., 2013). Oral administration of AQ delivered in a vehicle, such as peanut butter, is less invasive than other methods (such as viral delivery, direct infusion or gavage), and an oral delivery system could generalize to human studies.

This example demonstrates that oral administration of AQ protects neurons from oxygen glucose deprivation-induced cell death.

### Methods

*Animals.* 92 male F344 adult rats were used. Rats were kept on a 14/10-hr day/night cycle with access to food and water *ad libitum.* Weight for each animal was recorded two times per week, as to account for significant weight increases and/or decreases.

***Drugs.*** Apoaequorin (AQ; Quincy Bioscience) was prepared in double deionized water at a concentration of 7.4%. Experimental groups in the dose dependent experiments (n = 18) received 0 (n = 4), 3.6 (n = 5), 48 (n = 4), 240 (n = 3), or 480 mg/kg of AQ mixed into their daily PB. For the remainder of the studies, rats (*n* = 73) received 48 mg/kg of AQ mixed into their daily PB. Animals were assigned to one of five groups; No AQ (n = 12), 1 hour AQ (n = 17), 1 day AQ (n = 15), 2 days AQ (n = 15), and 7 days AQ (n = 14. Rats received ¼ teaspoon of PB placed in a petri dish in the cage every day at a designated time. Petri dishes were not removed until all PB was consumed. Animals were weighed twice per week, as to maintain proper AQ dosage.

AQ for infusion studies was prepared as previously described (Detert et al., 2013). IL-10 neutralizing antibody (nAb) and its IgG control were prepared in sterile PBS. 0.5 ug was infused at a rate of 1 ul/min through 1 ul Hamilton Syringes.

***Oxygen-Glucose Deprivation.*** On the last day of administration, rats were allowed 1 hour after PB consumption for digestion, deeply anesthetized with isoflurane, and coronal slices (400 µm) of dorsal hippocampus (dhpc; Bregma ⁻3.14 - ⁻4.16; Paxinos & Watson, 1998) were prepared using standard procedures (Moyer & Brown, 2007). Following 1 hr slice recovery in aCSF, one hemisphere of each brain (counterbalanced) was subjected to *in vitro* ischemia by transferring slices to an oxygen-glucose deprivation chamber (glucose replaced with fructose and bubbled with 95% N₂ - 5% CO₂ instead of a 95% O₂ - 5% CO₂) for 5 min, while the other hemisphere remained in recovery. All slices were then placed into oxygenated aCSF containing 0.2% trypan blue for a 30 min reperfusion period. Trypan blue stains dead cells while leaving living cells unstained (DeRenzis & Schechtman, 1973). The slices were rinsed twice in oxygenated, room temperature aCSF then fixed in 10% neutral buffered formalin overnight in the refrigerator. Slices were then cryoprotected in 30% sucrose, sectioned on a cryostat (40 µm), and mounted onto subbed slides for cell counts.

***Cell Counts.*** Slices were examined under an Olympus microscope (equipped with a digital camera) at 10X, and pictures were taken (CellSens). Trypan blue stained neurons within CA1 (about an 800 µm section) were counted by an experimenter blind to experimental conditions. Statistical analyses were performed using SPSS (v 21.0.0; IBM Corporation; Armonk, NY). An ANOVA was used to evaluate a drug effect, and Fisher's LSD post-hoc evaluations were used to evaluate group interactions. Asterisk (^{∗}) indicates p < .05.

***Western Blots.*** Animals were deeply anesthetized with isoflurane, brains rapidly removed, frozen, and stored at -80°C. Upon time of dissection, samples were dissected from dhpc (Bregma ⁻3.14 - ⁻4.16mm). Samples were homogenized, centrifuged at 4000 RPM for 20 min, supernatant was removed, and protein was measured using a Bradford protein assay kit (Bio-Rad). Protein samples were normalized and loaded for SDS-PAGE (12%). Proteins (30µg) were transferred onto PVDF membranes using the Turbo Transfer System (Bio-Rad). Membranes were incubated in blocking buffer (2 hr), primary antibody (overnight at 4 °C; 1:1000 mouse anti-aequorin [Chemicon] or 1:1000 rabbit anti-β-actin [Cell Signaling Technology], and secondary antibody (90 min; 1:20,000 goat anti-mouse [Santa Cruz Biotechnology] or 1:40,000 goat anti-rabbit [Millipore]). Membranes were then washed, placed in a chemiluminescence solution (Thermo Scientific), and imaged with a Syngene GBox. Images were taken with GeneSys software (v 1.2.4.0; Synoptics camera 4.2MP), and fluorescence for each band was evaluated with GeneTools software (v 4.02; Cambridge, England). Values are expressed as a percentage of control animals. Statistics were performed with SPSS (v. 21).

### Summary

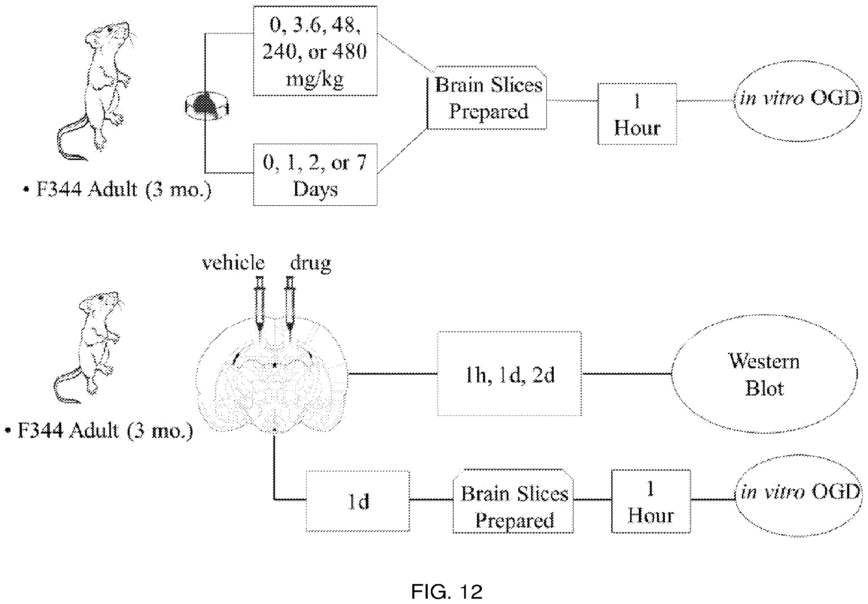

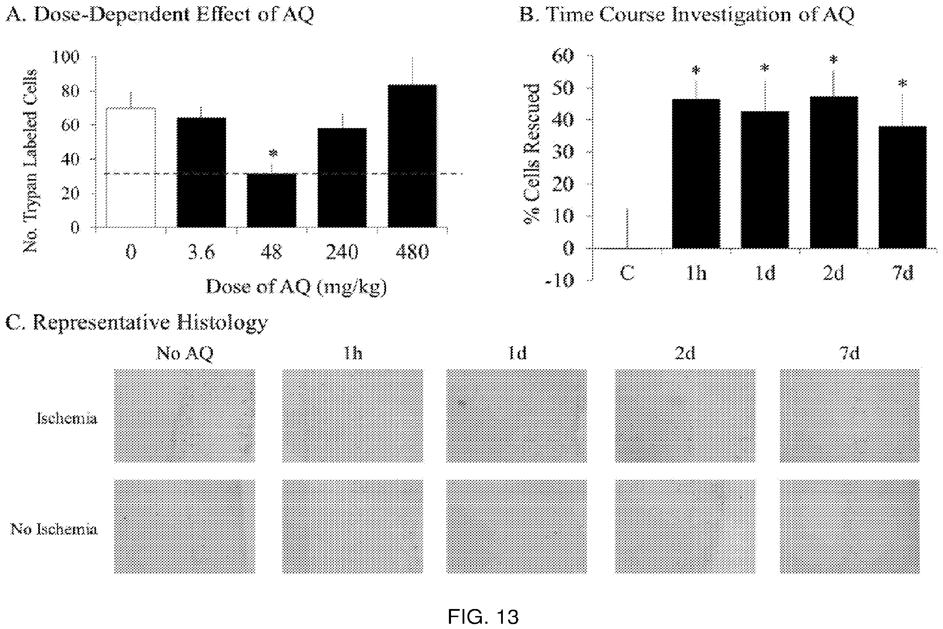

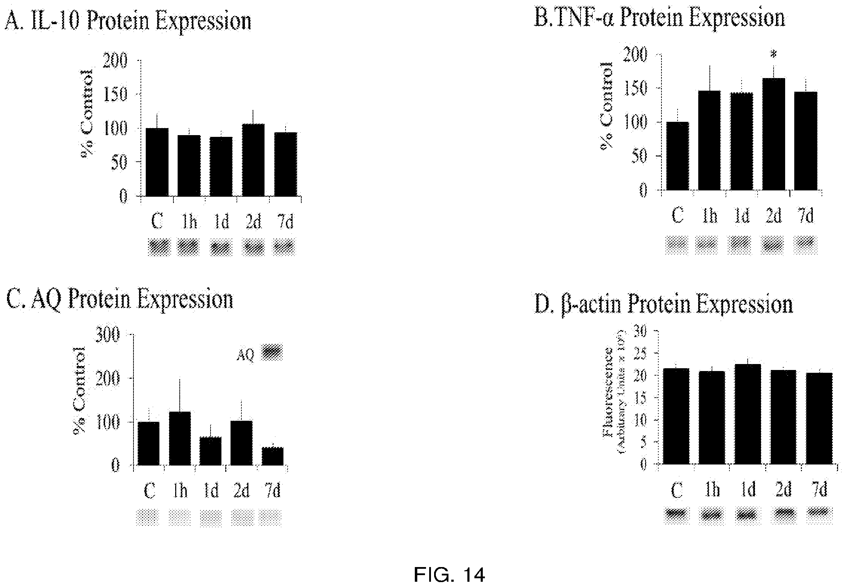

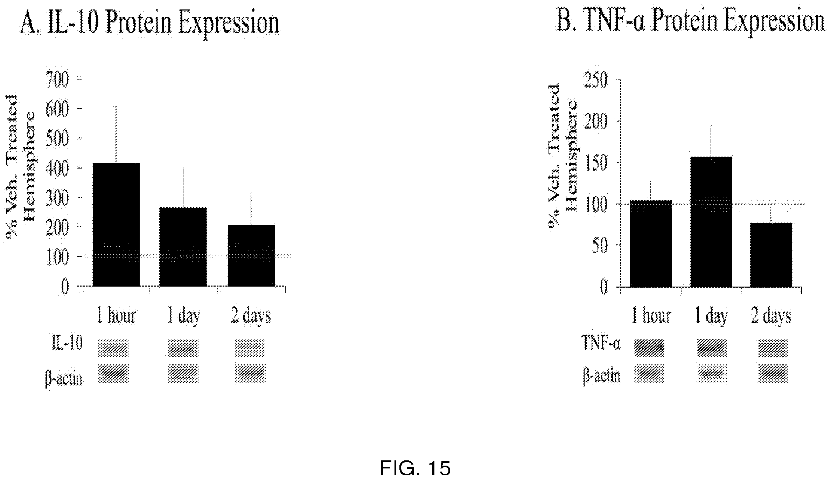

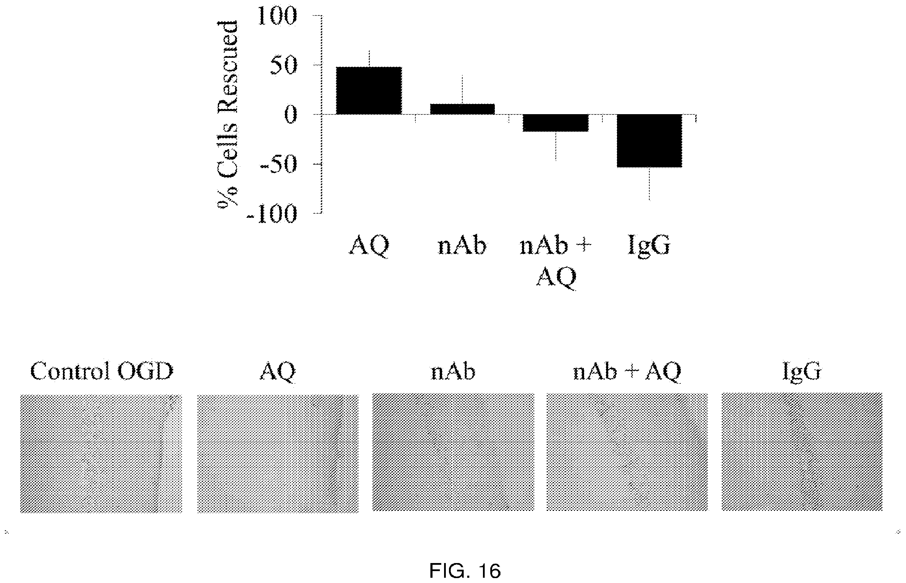

Figs. 12, 13A-C, 14A-D, 15A-B and 16 support the following conclusions: Apoaequorin's neuroprotective effect is dose-dependent. When administered orally, AQ protects from OGD-induced cell death at a dose of 48 mg/kg.

Apoaequorin has a long-lasting neuroprotective effect. Brain slices from rats that received 1 hour, 1 day, 2 days, or 7 days oral administration of AQ exhibited neuroprotection.

Apoaequorin administration alters cytokine protein expression.

TNF-α protein expression increases after 2 days oral administration of AQ, whereas IL-10 protein expression remains the same.

When infused, IL-10 protein expression increases at 1 hour as compared to vehicle infused hemisphere. Moreover, TNF-α increases at 1 day and thereafter protein levels dip below baseline. 4. Apoaequorin's neuroprotective effect is reversed by IL-10 neutralizing antibody.

When infused 1 day prior to in vitro OGD, AQ's neuroprotective effect is abolished when paired with an IL-10 nAb.

The present data suggest that AQ's neuroprotective effect involves IL-10; whether it be via neutralization of IL-10, or downstream cascades.

It is understood that the examples and embodiments described herein are for illustrative purposes only.

### REFERENCES

1. Go AS, Mozaffarian D, Roger VL, Benjamin EJ, Berry JD, et al. (2013) Executive summary: heart disease and stroke statistics--2013 update: a report from the American Heart Association. Circulation 127: 143-152.
2. Jones TA, Allred RP, Adkins DL, Hsu JE, O'Bryant A, et al. (2009) Remodeling the brain with behavioral experience after stroke. Stroke 40: S136-138.
3. Ginsberg MD (2009) Current status of neuroprotection for cerebral ischemia: synoptic overview. Stroke 40: S111-114.
4. Lin RC, Scheller RH (2000) Mechanisms of synaptic vesicle exocytosis. Ann Rev Cell Dev Biol 16: 19-49.
5. Berridge MJ (1998) Neuronal calcium signaling. Neuron 21: 13-26.
6. Bano D, Young KW, Guerin CJ, Lefeuvre R, Rothwell NJ, et al. (2005) Cleavage of the plasma membrane Na+/Ca2+ exchanger in excitotoxicity. Cell 120: 275-285.
7. Choi DW (1992) Excitotoxic cell death. J Neurobiol 23: 1261-1276.
8. Lee JM, Zipfel GJ, Choi DW (1999) The changing landscape of ischaemic brain injury mechanisms. Nature 399: A7-14.
9. Kristian T, Siesjo BK (1998) Calcium in ischemic cell death. Stroke 29: 705-718.
10. Baimbridge KG, Celio MR, Rogers JH (1992) Calcium-binding proteins in the nervous system. Trends Neurosci 15: 303-308.
11. Chard PS, Jordan J, Marcuccilli CJ, Miller RJ, Leiden JM, et al. (1995) Regulation of excitatory transmission at hippocampal synapses by calbindin D28k. Proc Natl Acad Sci U S A 92: 5144-5148.
12. Lledo PM, Somasundaram B, Morton AJ, Emson PC, Mason WT (1992) Stable transfection of calbindin-D28k into the GH3 cell line alters calcium currents and intracellular calcium homeostasis. Neuron 9: 943-954.
13. Celio MR, Pauls TL, Schwaller B (1996) Introduction to EF-hand calcium-binding proteins. In: Celio MR, editor. Guidebook to the Calcium-binding Proteins. New York: Oxford University Press. pp. 15-20.
14. Freund TF, Buzsaki G, Leon A, Baimbridge KG, Somogyi P (1990) Relationship of neuronal vulnerability and calcium binding protein immunoreactivity in ischemia. Exp Brain Res 83: 55-66.
15. Gary DS, Sooy K, Chan SL, Christakos S, Mattson MP (2000) Concentrationand cell type-specific effects of calbindin D28k on vulnerability of hippocampal neurons to seizure-induced injury. Brain Res Mol Brain Res 75: 89-95.
16. Iacopino AM, Christakos S, German D, Sonsalla PK, Altar CA (1992) Calbindin-D28K-containing neurons in animal models of neurodegeneration: possible protection from excitotoxicity. Brain Res Mol Brain Res 13: 251-261.
17. Rami A, Rabie A, Thomasset M, Krieglstein J (1992) Calbindin-D28K and ischemic damage of pyramidal cells in rat hippocampus. J Neurosci Res 31: 89-95.
18. Choi JH, Lee CH, Yoo KY, Hwang IK, Lee IS, et al. (2010) Age-related changes in calbindin-D28k, parvalbumin, and calretinin immunoreactivity in the dog main olfactory bulb. Cell Mol Neurobiol 30: 1-12.
19. De Jong GI, Naber PA, Van der Zee EA, Thompson LT, Disterhoft JF, et al. (1996) Age-related loss of calcium binding proteins in rabbit hippocampus. Neurobiol Aging 17: 459-465.
20. Moyer JR, Jr., Furtak SC, McGann JP, Brown TH (2011) Aging-related changes in calcium-binding proteins in rat perirhinal cortex. Neurobiol Aging 32: 1693-1706.
21. Bu J, Sathyendra V, Nagykery N, Geula C (2003) Age-related changes in calbindin-D28k, calretinin, and parvalbumin-immunoreactive neurons in the human cerebral cortex. Exp Neurol 182: 220-231.
22. Krzywkowski P, Potier B, Billard JM, Dutar P, Lamour Y (1996) Synaptic mechanisms and calcium binding proteins in the aged rat brain. Life Sci 59: 421-428.
23. Villa A, Podini P, Panzeri MC, Racchetti G, Meldolesi J (1994) Cytosolic Ca2+ binding proteins during rat brain ageing: loss of calbindin and calretinin in the hippocampus, with no change in the cerebellum. Eur J Neurosci 6: 1491-1499.
24. Mattson MP, Magnus T (2006) Ageing and neuronal vulnerability. Nat Rev Neurosci 7: 278-294.
25. Iacopino AM, Christakos S (1990) Specific reduction of calcium-binding protein (28-kilodalton calbindin-D) gene expression in aging and neurodegenerative diseases. Proc Natl Acad Sci U S A 87: 4078-4082.
26. Thibault O, Gant JC, Landfield PW (2007) Expansion of the calcium hypothesis of brain aging and Alzheimer's disease: minding the store. Aging Cell 6: 307-317.
27. Hof PR, Morrison JH (1991) Neocortical neuronal subpopulations labeled by a monoclonal antibody to calbindin exhibit differential vulnerability in Alzheimer's disease. Exp Neurol 111: 293-301.
28. Mikkonen M, Alafuzoff I, Tapiola T, Soininen H, Niettinen R (1999) Subfield- and layer-specific changes in parvalbumin, calretinin and calbindin-D28K immunoreactivity in the entorhinal cortex in Alzheimer's disease. Neuroscience 92: 515-532.
29. Sutherland MK, Wong L, Somerville MJ, Yoong LKK, Bergeron C, et al. (1993) Reduction of calbindin-28k mRNA levels in Alzheimer as compared to Huntington hippocampus. Brain Res Mol Brain Res 18: 32-42.
30. Yenari MA, Minami M, Sun GH, Meier TJ, Kunis DM, et al. (2001) Calbindin d28k overexpression protects striatal neurons from transient focal cerebral ischemia. Stroke 32: 1028-1035.
31. Fan Y, Shi L, Gu Y, Zhao Y, Xie J, et al. (2007) Pretreatment with PTDcalbindin D 28k alleviates rat brain injury induced by ischemia and reperfusion. J Cereb Blood Flow Metab 27: 719-728.
32. Urra X, Chamorro A (2013) Emerging issues in acute ischemic stroke. J Neurol 260: 1687-1692.
33. Khachaturian ZS (1984) Towards theories of brain aging. In: Kay DWK, Burrows GD, editors. Handbook of Studies on Psychiatry and Old Age. New York: Elsevier. pp. 7-30.
34. Khachaturian ZS (1989) The role of calcium regulation in brain aging: reexamination of a hypothesis. Aging 1: 17-34.
35. Landfield PW (1987) 'Increased calcium-current' hypothesis of brain aging. Neurobiol Aging 8: 346-347.
36. Wu WW, Oh MM, Disterhoft JF (2002) Age-related biophysical alterations of hippocampal pyramidal neurons: implications for learning and memory. Ageing Res Rev 1: 181-207.
37. Disterhoft JF, Oh MM (2007) Alterations in intrinsic neuronal excitability during normal aging. Aging Cell 6: 327-336.
38. Brait VH, Arumugam TV, Drummond GR, Sobey CG (2012) Importance of T lymphocytes in brain injury, immunodeficiency, and recovery after cerebral ischemia. J Cereb Blood Flow Metab 32: 598-611.
39. Mahesh VB, Dhandapani KM, Brann DW (2006) Role of astrocytes in reproduction and neuroprotection. Mol Cell Endocrinol 246: 1-9.
40. Sama DM, Norris CM (2013) Calcium dysregulation and neuroinflammation: Discrete and integrated mechanisms for age-related synaptic dysfunction. Ageing Res Rev.
41. Simon RP, Swan JH, Griffiths T, Meldrum BS (1984) Blockade of N-methyl-D-aspartate receptors may protect against ischemic damage in the brain. Science 226: 850-852.
42. Choi DW (1985) Glutamate neurotoxicity in cortical cell culture is calcium dependent. Neurosci Lett 58: 293-297.
43. Lysko PG, Cox JA, Vigano MA, Henneberry RC (1989) Excitatory amino acid neurotoxicity at the N-methyl-D-aspartate receptor in cultured neurons: pharmacological characterization. Brain Res 499: 258-266.
44. Park CK, Nehls DG, Graham DI, Teasdale GM, McCulloch J (1988) The glutamate antagonist MK-801 reduces focal ischemic brain damage in the rat. Ann Neurol 24: 543-551.
45. Nikonenko I, Bancila M, Bloc A, Muller D, Bijlenga P (2005) Inhibition of T-type calcium channels protects neurons from delayed ischemia-induced damage. Mol Pharmacol 68: 84-89.
46. Hadley MN, Zabramski JM, Spetzler RF, Rigamonti D, Fifield MS, et al. (1989) The efficacy of intravenous nimodipine in the treatment of focal cerebral ischemia in a primate model. Neurosurgery 25: 63-70.
47. Uematsu D, Araki N, Greenberg JH, Sladky J, Reivich M (1991) Combined therapy with MK-801 and nimodipine for protection of ischemic brain damage. Neurology 41: 88-94.
48. Lipton SA (2004) Failures and successes of NMDA receptor antagonists: molecular basis for the use of open-channel blockers like memantine in the treatment of acute and chronic neurologic insults. NeuroRx 1: 101-110.
49. Toma S, Chong KT, Nakagawa A, Teranishi K, Inouye S, et al. (2005) The crystal structures of semi-synthetic aequorins. Protein Sci 14: 409-416.
50. Cobbold PH, Lee JAC (1991) Aequorin measurements of cytoplasmic free calcium. In: McCormack JG, Cobbold PH, editors. Cellular calcium: a practical approach. New York: Oxford University Press. pp. 55-81.
51. Shimomura O, Inouye S (1996) Titration of recombinant aequorin with calcium chloride. Biochem Biophys Res Commun 221: 77-81.
52. Raley-Susman KM, Lipton P (1990) In vitro ischemia and protein synthesis in the rat hippocampal slice: the role of calcium and NMDA receptor activation. Brain Res 515: 27-38.
53. Newman GC, Hospod FE, Wu P (1989) Glucose utilization of ischemic hippocampal slices. J Neurosci Methods 28: 23-34.
54. Taylor CP, Burke SP, Weber ML (1995) Hippocampal slices: glutamate overflow and cellular damage from ischemia are reduced by sodium-channel blockade. J Neurosci Methods 59: 121-128.
55. Whittingham TS, Lust WD, Passonneau JV (1984) An in vitro model of ischemia: metabolic and electrical alterations in the hippocampal slice. J Neurosci 4: 793-802.
56. Pohorecki R, Becker GL, Reilly PJ, Landers DF (1990) Ischemic brain injury in vitro: protective effects of NMDA receptor antagonists and calmidazolium. Brain Res 528: 133-137.
57. Lipton P (1999) Ischemic cell death in brain neurons. Physiol Rev 79: 1431-1568.
58. Kirino T, Sano K (1984) Selective vulnerability in the gerbil hippocampus following transient ischemia. Acta Neuropathol 62: 201-208.
59. Moran DL, Marone PA, Bauter MR, Soni MG (2013) Safety assessment of Apoaequorin, a protein preparation: Subchronic toxicity study in rats. Food Chem Toxicol 57C: 1-10.
60. Moyer JR, Jr., Brown TM (2007) Visually-guided patch-clamp recordings in brain slices. In: Walz W, editor. Advanced Techniques for Patch-Clamp Analysis. 3rd ed. Totowa, NJ: Humana Press. pp. 169-227.
61. Moyer JR, Jr., Brown TH (1998) Methods for whole-cell recording from visually preselected neurons of perirhinal cortex in brain slices from young and aging rats. J Neurosci Methods 86: 35-54.
62. DeRenzis FA, Schechtman A (1973) Staining by neutral red and trypan blue in sequence for assaying vital and nonvital cultured cells. Stain Technol 48: 135-136.
63. Pfaffl MW (2001) A new mathematical model for relative quantification in real-time RT-PCR. Nucleic Acids Res 29: e45.
64. Simon RP, Niiro M, Gwinn R (1993) Prior ischemic stress protects against experimental stroke. Neurosci Lett 163: 135-137.
65. Xu GP, Dave KR, Vivero R, Schmidt-Kastner R, Sick TJ, et al. (2002) Improvement in neuronal survival after ischemic preconditioning in hippocampal slice cultures. Brain Res 952: 153-158.
66. Oh MM, Oliveira FA, Disterhoft JF (2010) Learning and aging related changes in intrinsic neuronal excitability. Front Aging Neurosci 2: 2.
67. Pera J, Zawadzka M, Kaminska B, Szczudlik A (2004) Influence of chemical and ischemic preconditioning on cytokine expression after focal brain ischemia. J Neurosci Res 78: 132-140.
68. Mattson MP, Rychlik B, Chu C, Christakos S (1991) Evidence for calciumreducing and excito-protective roles for the calcium-binding protein calbindin-D28k in cultured hippocampal neurons. Neuron 6: 41-51.
69. Lowenstein DH, Gwinn RP, Seren MS, Simon RP, McIntosh TK (1994) Increased expression of mRNA encoding calbindin-D28K, the glucose-regulated proteins, or the 72 kDa heat-shock protein in three models of acute CNS injury. Brain Res Mol Brain Res 22: 299-308.
70. Mattson MP, Cheng B, Baldwin SA, Smith-Swintosky VL, Keller J, et al. (1995) Brain injury and tumor necrosis factors induce calbindin D-28k in astrocytes: evidence for a cytoprotective response. J Neurosci Res 42: 357-370.
71. Hwang IK, Kang TC, Lee JC, Park SK, An SJ, et al. (2003) Chronological alterations of calbindin D-28k immunoreactivity in the gerbil main olfactory bulb after ischemic insult. Brain Res 971: 250-254.
72. Freund TF, Ylinen A, Miettinen R, Pitkanen A, Lahtinen H, et al. (1992) Pattern of neuronal death in the rat hippocampus after status epilepticus. Relationship to calcium binding protein content and ischemic vulnerability. Brain Res Bull 28: 27-38.
73. Klapstein GJ, Vietla S, Lieberman DN, Gray PA, Airaksinen MS, et al. (1998) Calbindin-D28k fails to protect hippocampal neurons against ischemia in spite of its cytoplasmic calcium buffering properties: evidence from calbindin-D28k knockout mice. Neuroscience 85: 361-373.
74. Tymianski M, Wallace MC, Spigelman I, Uno M, Carlen PL, et al. (1993) Cell-permeant Ca2+ chelators reduce early excitotoxic and ischemic neuronal injury in vitro and in vivo. Neuron 11: 221-235.
75. Tymianski M, Spigelman I, Zhang L, Carlen PL, Tator CH, et al. (1994) Mechanism of action and persistence of neuroprotection by cell-permeant Ca2+ chelators. J Cereb Blood Flow Metab 14: 911-923.
76. Abdel-Hamid KM, Baimbridge KG (1997) The effects of artificial calcium buffers on calcium responses and glutamate-mediated excitotoxicity in cultured hippocampal neurons. Neuroscience 81: 673-687.
77. Cronberg T, Rytter A, Wieloch T (2005) Chelation of intracellular calcium reduces cell death after hyperglycemic in vitro ischemia in murine hippocampal slice cultures. Brain Res 1049: 120-127.
78. Dubinsky JM (1993) Effects of calcium chelators on intracellular calcium and excitotoxicity. Neurosci Lett 150: 129-132.
79. Barone FC, White RF, Spera PA, Ellison J, Currie RW, et al. (1998) Ischemic preconditioning and brain tolerance: temporal histological and functional outcomes, protein synthesis requirement, and interleukin-1 receptor antagonist and early gene expression. Stroke 29: 1937-1950; discussion 1950-1931.
80. Kirino T (2002) Ischemic tolerance. J Cereb Blood Flow Metab 22: 1283-1296.
81. Jung M, Sola A, Hughes J, Kluth DC, Vinuesa E, et al. (2012) Infusion of IL-10-expressing cells protects against renal ischemia through induction of lipocalin-2. Kidney Int 81: 969-982.
82. Levin SG, Godukhin OV (2011) Anti-inflammatory cytokines, TGF-beta1 and IL-10, exert anti-hypoxic action and abolish posthypoxic hyperexcitability in hippocampal slice neurons: comparative aspects. Exp Neurol 232: 329-332.
83. Lin RC, Scheller RH (2000) Mechanisms of synaptic vesicle exocytosis. Annu Rev Cell Dev Biol 16: 19-49.
84. Hasko G, Szabo C, Nemeth ZH, Lendvai B, Vizi ES (1998) Modulation by dantrolene of endotoxin-induced interleukin-10, tumour necrosis factor-alpha and nitric oxide production in vivo and in vitro. Br J Pharmacol 124: 1099-1106.
85. Wang X, Li X, Erhardt JA, Barone FC, Feuerstein GZ (2000) Detection of tumor necrosis factor-alpha mRNA induction in ischemic brain tolerance by means of real-time polymerase chain reaction. J Cereb Blood Flow Metab 20: 15-20.
86. Wang X, Li X, Currie RW, Willette RN, Barone FC, et al. (2000) Application of real-time polymerase chain reaction to quantitate induced expression of interleukin-1beta mRNA in ischemic brain tolerance. J Neurosci Res 59: 238-246.
87. Ohtsuki T, Ruetzler CA, Tasaki K, Hallenbeck JM (1996) Interleukin-1 mediates induction of tolerance to global ischemia in gerbil hippocampal CA1 neurons. J Cereb Blood Flow Metab 16: 1137-1142.
88. Mallard C, Hagberg H (2007) Inflammation-induced preconditioning in the immature brain. Semin Fetal Neonatal Med 12: 280-286.
89. Gerard C, Bruyns C, Marchant A, Abramowicz D, Vandenabeele P, et al. (1993) Interleukin 10 reduces the release of tumor necrosis factor and prevents lethality in experimental endotoxemia. J Exp Med 177: 547-550.
90. Spera PA, Ellison JA, Feuerstein GZ, Barone FC (1998) IL-10 reduces rat brain injury following focal stroke. Neurosci Lett 251: 189-192.
91. Yang J, Ahn HN, Chang M, Narasimhan P, Chan PH, et al. (2013) Complement component 3 inhibition by an antioxidant is neuroprotective after cerebral ischemia and reperfusion in mice. J Neurochem 124: 523-535.
92. Hwang IK, Yoo KY, Kim DW, Lee HJ, Kang HY, et al. (2006) Transient ischemia-induced changes of interleukin-2 and its receptor beta immunoreactivity and levels in the gerbil hippocampal CA1 region. Brain Res 1106: 197-204.
93. Ishikawa M, Vowinkel T, Stokes KY, Arumugam TV, Yilmaz G, et al. (2005) CD40/CD40 ligand signaling in mouse cerebral microvasculature after focal ischemia/reperfusion. Circulation 111: 1690-1696.
94. Tan J, Town T, Mori T, Obregon D, Wu Y, et al. (2002) CD40 is expressed and functional on neuronal cells. EMBO J 21: 643-652.

## Claims

1. Apoaequorin for use in a method of therapy comprising preconditioning neurons to reduce neuronal cell death following ischemia in a subject susceptible to ischemic cell death, said apoaequorin provided in a therapeutically effective dosage to precondition neurons in the subject to reduce neuronal cell death and wherein said apoaequorin is administered orally in the form of a nutraceutical composition.

2. Apoaequorin for use according to claim 1 wherein the apoaequorin is in a unit dosage form selected from a tablet or capsule.

## Patentansprüche

1. Apoaequorin zur Verwendung in einem Therapieverfahren, das die Vorkonditionierung von Neuronen zur Verringerung des neuronalen Zelltods nach Ischämie in einem für ischämischen Zelltod anfälligen Subjekt umfasst,
wobei das Apoaequorin in einer therapeutisch wirksamen Dosierung bereitgestellt wird, um Neuronen in dem Subjekt vorzukonditionieren, um den neuronalen Zelltod zu reduzieren, und wobei das Apoaequorin oral in Form einer nutrazeutischen Zusammensetzung verabreicht wird.

2. Apoaequorin zur Verwendung nach Anspruch 1, wobei das Apoaequorin in einer Einheitsdosierungsform vorliegt, die aus einer Tablette oder Kapsel ausgewählt wird.

## Revendications

1. Apoaequorine pour une utilisation dans un procédé de thérapie comprenant le préconditionnement des neurones pour réduire la mort des cellules neuronales suite à une ischémie chez un sujet sensible à la mort cellulaire ischémique, ladite apoaequorine étant fournie en une dose thérapeutiquement efficace pour préconditionner les neurones chez le sujet afin de réduire la mort des cellules neuronales et dans laquelle ladite apoaequorine est administrée par voie orale sous la forme d'une composition nutraceutique.

2. Apoaequorine pour une utilisation selon la revendication 1, dans laquelle l'apoaequorine est sous une forme posologique unitaire choisie parmi un comprimé ou une capsule.
